# EUROPÄISCHE PATENTSCHRIFT

(11) **EP 2 482 912 B1**
(45) Veröffentlichungstag und Bekanntmachung des Hinweises auf die Patenterteilung: **23.10.2019**
(21) Anmeldenummer: 10739328.2
(22) Anmeldetag: 22.07.2010
(51) Int. Cl.: A61M 27/00, A61M 1/00, A61B 1/00, A61B 1/12, A61B 1/273, A61B 17/22, A61M 25/09, A61F 13/36, A61B 90/00

(54) **VAKUUMSCHWAMMDRAINAGE**
VACUUM SPONGE DRAINAGE
DRAINAGE PAR ÉPONGE SOUS VIDE

(30) Priorität: 30.09.2009 DE 102009043472; 09.12.2009 DE 102009057374; 29.03.2010 DE 102010013271; 30.03.2010 DE 102010013439; 01.04.2010 DE 102010013849; 01.04.2010 DE 102010013848
(43) Veröffentlichungstag der Anmeldung: 08.08.2012
(60) Teilanmeldung: 17189242.5
(73) Patentinhaber: Lohmann & Rauscher GmbH & Co. KG, 56567 Neuwied (DE)
(72) Erfinder: SCHORSCH, Tobias Paul Heinrich, Hamburg 20253 (DE); LOSKE, Gunnar, 22926 Ahrensburg (DE)
(74) Vertreter: Seranski, Klaus
(86) Internationale Anmeldenummer: PCT/EP2010/060671
(87) Internationale Veröffentlichungsnummer: WO 2011/038949

(56) Entgegenhaltungen:
- WO-A1-2004/041346
- WO-A2-03/028786
- WO-A2-2006/114637
- DE-A1-102008 061 535
- US-A- 6 123 697
- US-A1- 2007 219 497
- US-A1- 2007 282 310

## Beschreibung

Die Erfindung betrifft eine Vakuumschwammeinheit zur Verwendung im menschlichen oder tierischen Körper mit einem Fluidsammelelement, insbesondere einer Schwammeinheit, und einem Fluidkommunikationselement, insbesondere einem Drainageschlauch, welches(er) wenigstens teilweise in dem Fluidsammelelement angeordnet und fluidleitend mit dem Fluidsammelelement verbunden ist. Die Erfindung betrifft ferner ein Vakuumschwammsystem mit einer Vakuumschwammeinheit und einem Führungselement und/oder Versorgungselement, ein Verfahren zum Einführen des Vakuumschwammsystems in einen menschlichen oder tierischen Körper, ein Verfahren zum Herstellen einer Vakuumschwammeinheit, eine Verwendung eines fluidsammelnden Materials zur Herstellung einer Vakuumschwammeinheit, eine Verwendung eines Vakuumschwammsystems in einem menschlichen oder tierischen Körper und eine Verwendung einer Vakuumschwammeinheit als Saugeransatz.

WO 2004/041346 A1 beschreibt ein endoskopisches Wundpflegebehandlungssystem. Die WO 2004/041346 A1 bezieht sich insbesondere auf eine endoskopische Komponente, die die Anwendung der Unterdrucktherapie auch im Inneren eines menschlichen oder tierischen Körpers ermöglicht.

Der Erfindung liegt die Aufgabe zugrunde, eine Weiterentwicklung der WO 2004/041346 A1 bereitzustellen, die die Nachteile der bekannten Systeme beseitigt oder wenigstens reduziert.

Erfindungsgemäß wird die Aufgabe dadurch gelöst, dass das Fluidsammelelement einen Kanal zum Führen eines Führungsselements, insbesondere eines Führungsdrahts, und/oder eines Versorgungselements, insbesondere einer Sondeneinheit und/oder einem Endoskop, durch das Fluidsammelelement aufweist.

Der Erfindung liegt die Erkenntnis zugrunde, dass die Verwendung der Vorrichtung der WO 2004/041346 A1 in Bereichen innerhalb des menschlichen oder tierischen Körpers tiefer als ca. 10cm (von der Körperöffnung aus gesehen) im menschlichen oder tierischen Körper zu Anwendungsproblemen führt. Ferner hat der Erfinder erkannt, dass das Anwendungsgebiet der Vorrichtung der WO 2004/041346 A1 sich insbesondere auf nur einseitig offene Bereiche innerhalb des Körpers beschränkt und bei Verwendung in zweiseitig offenen, kanalartig ausgebildeten Bereichen innerhalb des Körpers problematisch ist.

Die Weiterentwicklung erreicht vorteilhaft, dass die erfindungsgemäße Vakuumschwammeinheit für Bereiche innerhalb des menschlichen oder tierischen Körpers geeignet ist, die insbesondere tiefer als 10cm (von der Körperöffnung aus gesehen) im menschlichen oder tierischen Körper gelegen sind, und dass die Vakuumschwammeinheit nicht nur in einseitig offenen Bereichen innerhalb des Körpers, sondern besonders vorteilhaft in zweiseitig offenen, kanalartig ausgebildeten Hohlräumen (insbesondere in dem gesamten Magen-Darm-Trakt) innerhalb des Körpers verwendet werden kann.

Der Erfindung liegt die Idee zugrunde, dass ein Kanal innerhalb des Fluidsammelelements, das insbesondere eine Schwammeinheit ist, diese und weitere Vorteile erreichen kann.

Der Kanal kann dazu genutzt werden, um ein Führungselement, insbesondere einen Führungsdraht (oder auch Sonde, Hülse, Stab, Endoskop), durch den Kanal zu führen, um das Fluidsammelelement entlang des Führungselementes zu führen und zu positionieren. In der WO 2004/041346 A1 wird die Positionierung dadurch erreicht, dass sich das Fluidsammelmittel am Ausgang der inneren Hülse entfaltet, was den Nachteil hat, dass nur Bereiche innerhalb des Körpers versorgt werden können, die mit der inneren Hülse erreicht werden. Die Erfindung behebt diesen Nachteil, indem die Positionierung des Fluidsammelelements in entlegenere und verwinkelte Körperbereiche mittels eines im Kanal geführten Führungselementes ermöglicht wird.

Der Kanal kann ergänzend oder alternativ dazu genutzt werden, um ein Versorgungselement in den Körper einzuführen. Die Vorrichtung der WO 2004/041346 A1 bietet den Nachteil, dass wenn sie im gesamten Magen-Darm-Trakt verwendet werden würde, eine Ernährung oder Darmleerung nur nach Entfernen des Fluidsammelmittels möglich wäre. Erfindungsgemäß wird dieser Nachteil dadurch beseitigt, dass der Kanal ein Versorgungselement beispielsweise zum Ernähren, Darmentleeren, Entgasen und/oder zur Spülung von oral oder aboral des Schwammes gelegenen Darmabschnitten aufnehmen kann, so dass das Fluidsammelelement während dieser Vorgänge nicht entfernt werden muss.

Der Kanal kann ergänzend oder alternativ dazu genutzt werden, um ein Versorgungselement in dem Körper angeordnet zu belassen. In der WO 2004/041346 A1 wird das Endoskop zunächst aus dem Körper entfernt und anschließend das Fluidsammelmittel eingebracht. Eine optische Überwachung mittels des Endoskops ist also nach Positionierung und Aktivierung des Fluidsammelmittels nicht mehr bzw. nur sehr aufwendig möglich. Die Erfindung behebt diesen Nachteil, indem der Kanal Platz für beispielsweise ein Endoskop bietet, so dass eine gleichzeitige Nutzung von Fluidsammelelement und Endoskop möglich ist. Auch ist es möglich, bei oder nach Aktivierung des Fluidsammelelementes beispielsweise ein Endoskop vorzusehen/einzuführen, um den Zustand des zu versorgenden Körperbereichs zu überwachen und die Platzierung zu kontrollieren..

Das Fluidsammelelement ist besonders bevorzugt eine Schwammeinheit. Die Schwammeinheit erfüllt die Funktion eines Ab- und Zuleitens von Flüssigkeiten und Gasen in und aus dem Körper. Dies ist insbesondere die Funktion einer Druckverteilung, so dass ein an das Fluidkommunikationselement angelegter Unterdruck ein Kollabieren der Schwammeinheit und des diese umgebenden Körperbereichs veranlasst.

Bevorzugt bezieht sich die Erfindung auf ein Einführsystem zum Positionieren einer Schwammdrainage in einem Intestinallumen, einem Hohlorgan, einer Körperhöhle oder einem Gewebeabszess, wobei das System umfasst: einen Führungsdraht, einen mit einem Drainageschlauch verbundenen Polyurethanschwamm, welche sich mit einer Positionierungshülse in einer Führungshülse befinden, wobei der Polyurethanschwamm eine Röntgenmarkierung besitzt. Das Einführsystem ist bevorzugt für eine Schwammdrainage zur endocavitären (bzw. intracavitären) oder endoluminalen (bzw. intraluminalen) Vakuumschwammtherapie ausgestaltet.

Durch die Einlage eine Drainage verbunden mit einem offenporigen Polyurethanschwamm lassen sich bekanntermaßen innere Wunden bei einer postoperativen Anastomoseninsuffizienz am Enddarm behandeln. Die Drainage wird hierzu unter Sog gesetzt, sie entfernt das Wundsekret, führt zur Wundverklebung und Abheilung. Durch das bislang verfügbare Einführsystem lassen sich nur körperöffnungsnahe Wunden versorgen.

Mit der Erfindung zu erzielenden Vorteile liegen in der tieferen Positionierung einer Schwammdrainage und der sich hierdurch ergebenden erweiterten Indikationsstellung der Vakuumschwammtherapie. Das Schwammsystem lässt sich mit der Erfindung auch in Speiseröhre, Magen, Zwölffingerdarm, Dickdarm sowie in alle Körperhöhlen oder Wundhöhlen einlegen, die mit einem Endoskop erreicht werden können. Durch eine Positionierung in einem Darmlumen kann ein kompletter Verschluss desselben erzielt werden. Es kann ebenso gleichzeitig eine Drainage und ein innerer Verschluss von inneren Wunden erfolgen und diese hierdurch zur Abheilung gebracht werden. Eine Speiseröhrenperforation kann endoskopisch durch die Versorgung mittels eines gecoverten selbstexpandierenden Stents erfolgen. Dieser wird mit einem Einführsystem über einen zuvor endoskopisch eingelegten Führungsdraht über dem Defekt eingebracht und soll diesen von innen abdichten. Hierzu ist auch eine von außen eingelegte Wunddrainage notwendig, Mit der Erfindung wird über einen endoskopisch eingelegten Führungsdraht das Einführsystem positioniert und ein offenporiger Polyurethanschwamm, welcher mit einer Drainage verbunden ist, über dem Perforationdefekt der Speiseröhre eingelegt. Die Drainage wird mit einer Vakuumpumpe unter einen Unterdruck gesetzt. Hierdurch kollabiert das Speiseröhrenlumen über dem Schwamm, der Defekt wird verschlossen und Wundsekret gleichzeitig drainiert. Der Vorteil dieses Verfahrens gegenüber dem Stentsystem ist die Drainage des Wundsekretes nach innen und die Ableitung über die Drainage durch die Sogwirkung in Richtung des Darmlumens. Dieses ist insbesondere wirkungsvoll an Organen die natürlicherweise unter einem Unterdruck stehen (Organe in der Brusthöhle) oder Wunden und Wundhöhlen die mit der Brusthöhle in Kontakt stehen und sich hier durch physiologischerweise unter einem Unterdruck befinden. Durch den Vakuumsog, der über die Drainage an den offenporigen Polyurethanschwamm angelegt wird, kann sich das Wundsekret über die Drainage entleeren und wird nicht entlang des physiologischerseits bestehenden Druckgradienten und bei Atembewegungen in Richtung des Brustkorbes gezogen.

Bevorzugt bezieht sich die Erfindung auf einen geschlitzten Overtube (Schutzhülse) zum Einführen und Wechseln von Endoskopen und medizinischen Instrumenten in ein Intestinallumen, ein Hohlorgan, eine Körperhöhle oder Gewebeabszess sowohl über natürliche als auch künstliche Körperöffnungen.

Bei der endoskopischen Untersuchung des Magendarmtraktes, insbesondere der Speiseröhre und des Magen, aber auch des Dünndarmes werden Schutzhülsen, sogenannte Overtubes benutzt. Sie werden vor der Untersuchung über das Endoskop geschoben und nach dem Einführen des Endoskops in das Darmlumen über dem liegenden Endoskop in den Darm nachgeführt. Overtubes dienen nach ihrer Platzierung der sicheren Überbrückung des Rachenraumes und der Speiseröhre bei endoskopischen Behandlungen. Insbesondere wenn das Einführen eines Endoskops im Rachenraum und der oberen Speiseröhre schwierig ist, und ein Endoskop bei der Untersuchung wiederholt ein- und ausgeführt werden muss, können Verletzungen des Rachens und der Speiseröhrenwand vermieden werden, da der Overtube als Schutzhülsenschienung für das Endoskop dient. Overtubes werden eingesetzt, um bei der endoskopischen Entfernung von Fremdkörpern Verletzungen der Darmwand zu verhindern. Bei der endoskopischen Untersuchung des Dünndarms dienen Overtubes als Schienung und Schutz bei der Überbrückung von Speiseröhre, Magen und Dünndarm sowie als technisches Hilfsmittel zur Erreichung tieferer Darmabschnitte. Die bislang verfügbaren Overtubes werden vor einer endoskopischen Untersuchung auf dem Endoskop aufgeschoben, anschließend wird über eine Körperöffnung eingespiegelt, der Overtube über dem Endoskop nachgeschoben und als Schienung platziert. Die endoskopische Untersuchung wird dann über dem liegenden Overtube vorgenommen. Flexible endoskopische Operationen werden derzeit über spezielle Instrumente ausgeführt, die über Arbeitskanäle des flexiblen Endoskops eingeführt werden. Bei endoskopischen Operationen mit starren Endoskopen, z. B bei einer Bauchhöhlenspiegelung, wird eine starre Geräteoptik über einen Trokar in eine Körperhöhle eingebracht und weitere zusätzliche starre Instrument über gesonderte Inzisionen über Trokare eingesetzt. Neuerdings werden zusammen mit der Geräteoptik Operationsinstrumente über einen einzelnen Zugang (Singleport) eingesetzt.

Erfindungsgemäß ist der Overtube so konstruiert, dass er über die gesamte Länge längsgeschlitzt ist. Die Erfindung kann im Durchmesser und Länge nach den erforderlichen Gegebenheiten adaptiert werden. Sie besteht im Prinzip aus einem biegsamen aber formstabilen Kunststoffschlauch. Die mit der Erfindung zu erzielenden Vorteile liegen in der Möglichkeit zu jedem Zeitpunkt einer endoskopischen Untersuchung einen Overtube auf ein Endoskop aufbringen und entfernen zu können. Der Overtube kann über dem Längsschlitz um das Endoskop angelegt werden, so dass das Endoskop in dem Overtube zu liegen kommt. Anschließend lässt sich der Overtube mit dem Endoskop als Schienung in eine Körperöffnung vorschieben. Die Entfernung des Overtubes ist bei einem im Körper einliegenden Endoskop durch seitliches Herausziehen über den Schlitz möglich. Durch die Erfindung ergeben sich zahlreiche neue endoskopische Behandlungsmöglichkeiten. Die Erfindung ermöglicht es, technisch einfach und sicher, mehrere medizinische Instrumente über eine Körperöffnung in den Körper einzuführen. So kann z.B. ein Gastroskop gemeinsam mit einem medizinischen Instrument in den Magen eingeführt werden. Dazu wird zunächst über dem, in dem Magen einbrachten, Gastroskop der Overtube aufgesetzt und in den Magen eingeschoben. Anschließend wird das Gastroskop entfernt, um dann gemeinsam mit diesem ein weiteres medizinisches Gerät (z. B eine Drainage oder eine semiflexible Zange) über den Overtube unter endoskopischer Sicht in den Magen einzuführen. Der Overtube lässt sich dann bei den im Magen platzierten Geräten zurückziehen und entfernen. Er wird dann wieder nur auf das Endoskop aufgesetzt und wieder bis in den Magen vorgeschoben. Dieses Platzierungsmanöver kann mehrfach wiederholt werden, so dass sich auf diese Art und Weise mehrere Instrumente mit einem Gastroskop gleichzeitig in den Magen einlegen lassen. Durch die Erfindung ist es möglich unter flexibler endoskopischer optischer Kontrolle mit semiflexiblen Instrumenten zu operieren. Die semiflexiblen Instrumente lassen sich wie beschrieben in den Magen einbringen. Das Endoskop dient als Optik für die Bedienung der semiflexiblen Instrumente und zur Zufuhr des Untersuchungsgases. Zur endoskopischen Operation kann der Overtube entfernt werden, um einen Gasverlust zu vermeiden und den Reibungswiderstand möglichst gering zuhalten. Es werden durch die Erfindung dieselben operativen Techniken, wie sie aus der starren operativen Endoskopie z. B der Bauchhöhlenspiegelung bekannt sind, möglich sein. Es wird auch möglich sein, sogenannte NOTES (Natural Orifice Transluminal Endoscopic Surgery) Verfahren durchzuführen. Beispielsweise wird unter flexibler endoskopischer optischer Kontrolle die Eröffnung der Magenwand zur Eröffnung der Bauchhöhle und speziellen operativen Maßnahmen mittels der eingeführten semiflexiblen Instrumente möglich sein. Bevorzugt wird der Overtube über eine schon eingeführte Sonde, Führungsdraht oder Instrument eingeführt.

Der Overtube kann auch zur Bergung von Polypen oder endoskopischen Operationen eingesetzt werden. So kann nach einer Polyektomie der Overtube auf das Endoskop seitlich aufgesetzt und bis zur Abtragungsstelle unter endoskopischer Sicht vorgeführt werden. Der Polyp kann dann mit dem Endoskop herausgezogen werden, der Overtube bleibt dabei im Darm liegen. Nach der Bergung des Polypen kann das Endoskop wieder über den liegenden Overtube eingeführt werden. Der Nutzung des Overtubes verringert die Untersuchungszeit und erhöht die Patientensicherheit, da bei dem erneuten Einführen des Endoskops bei liegendem Overtube der Darm nicht erneute endoskopiert werden muss, sondern der Overtube als Leit- bzw Führungsschiene genutzt werden kann, um an den Untersuchungsort zu kommen.

Bevorzugt bezieht sich die Erfindung auf einen Schwammsaugeransatz zum Absaugen von Körperflüssigkeiten, Wundsekreten, Spülflüssigkeiten, Gasen bestehend aus einem Drainagerohr, welches mit einem Fluidsammelmiltel verbunden ist. Bevorzugt besitzt das Fluidsammelmittel eine Röntgenmarkierung. Bevorzugt umfasst das Fluidsammelmittel eine elastische komprimierbare Struktur mit Fluidkanälen. Bevorzugt umfasst das Fluidsammelmittel einen offenporigen Polyurethanschwamm. Bevorzugt weist das Drainagerohr am distalen Ende seitliche Öffnungen auf. Bevorzugt besteht das Drainagerohr aus Kunststoff. Bevorzugt besteht das Drainagerohr aus Metall. Bevorzugt kann in das Drainagerohr wie in eine Hülse ein weiteres Drainagerohr bündig abschließend eingeführt werden, so dass hierüber die Saugung vorgenommen wird. Ferner bezieht sich die Erfindung auf ein Fluidsammelmittel, welches eine Öffnung besitzt, in die das Distalende eines Drainagerohrs eingeführt werden kann, so dass ein herkömmlicher Saugeransatz als ein Schwammsaugeransatz benutzt werden kann. Bevorzugt hat das Fluidsammelmittel eine Befestigungsmöglichkeit (Klebeband, Faden, Klettband), um es an dem Distalende eines Drainagerohrs zu befestigen.

Bei Operationen werden Wundsekret und Spülflüssigkeit üblicherweise mit einem Saugeransatz, welcher mit einem Absaugschlauch verbunden ist, entfernt. Der konventionelle Saugeransatz besteht aus einem Rohr, an dessen Distalende sich eine oder mehrere Perforationsöffnungen befinden. Der konventionelle Saugeransatz saugt sich häufig an Gewebeweichteilen fest, ebenso kommt es häufig zur Verstopfung der Perforationsöffnungen.

Die Erfindung kann bei sämtlichen Operationen, auch z. B. minimal-invasiven laparoskopischen, thorakoskopischen und konventionellen Abdominaloperationen angewandt werden. Die Erfindung kann auch in außermedizinischen Bereichen angewandt werden, wenn Flüssigkeiten entfernt werden sollen. An dem distalen Ende eines Drainagerohrs als Saugeransatz wird über den Perforationsöffnungen erfindungsgemäß ein offenporiger Polyurethanschwamm befestigt. Dieser Schwamm wirkt wie ein Filter, gleichzeitig verhindert er, dass sich der Saugeransatz an Weichteilgewebe festsaugen kann. Mit dem Schwamm können infektiöse Beläge, die den Weichteilen oder Organen aufliegen, abgewischt werden. Hierdurch kann eine effektive und schonende Reinigung vorgenommen werden. Insbesondere bei Operationen bei denen eine Infektion des Operationsgebietes (z.B. einer Bauchfellentzündung vorliegt), lässt sich die Erfindung gut nutzen. In engen Zwischenräumen und direktem Gewebekontakt, ist durch den offenporigen Schwamm eine Absaugung von Sekret möglich, die mit einem herkömmlichen Sauger bislang schlecht gelingt. Die Erfindung kann in verschiedenen Varianten gefertigt werden:
Variante 1: Über den Perforationsöffnungen am Distalende eines Saugeransatzes wird ein offenporiger Schwamm durch Ankleben, Naht oder andere Fixierungsmöglichkeiten befestigt. Der Schwamm wird hierzu zentral eingeschnitten oder besitzt eine zentrale Ausstanzung, in die das Distalende des Saugeransatzes eingeführt werden kann. Es ist möglich, einen bereits vorhanden Saugeransatz durch das Aufsetzen und Befestigen eines Schwammes in die Erfindung umzuwandeln.
Variante 2: Ein offenporiger Schwamm wird über dem Distalende einer Saugerhülse, welche am Distalende Perforationsöffnungen besitzt, befestigt. In diese Hülse kann ein Saugeransatz eingeführt werden. Die Hülse schließt am proximalen Ende mit dem Saugeransatz bündig ab, so dass bei der Saugung ein Vakuum am Schwamm aufgebaut werden kann. Diese Variante hat den Vorteil, dass man bei der Operation durch die Möglichkeit des Abziehens der Hülse, sowohl die Vorteile einer schwammarmierten Absaugung als auch im Wechsel die konventionelle direkte Absaugung mit dem Saugeransatz nutzen kann.

Bevorzugt bezieht sich die Erfindung auf eine Schwammdrainage zum endoskopischen, radiologischen oder operativen Einlegen in ein Intestinallumen, ein Hohlorgan, einer Körperhöhle oder einem Gewebeabszess, wobei die Schwammdrainage ein Führungselement und das Drainagesystem (ein mit einer Drainageleitung verbundenes Fluidsammelmittel) umfasst. Ferner bezieht sich die Erfindung auf ein Drainagesystem, wobei das Fluidsammelmittel eine Röntgenmarkierung besitzt. Bevorzugt umfasst das Fluidsammelmittel eine elastische komprimierbare Struktur mit Fluidkanälen, Bevorzugt umfasst das Fluidsammelmittel einen offenporigen Polyurethanschwamm. Bevorzugt ist das Führungselement ein Draht. Bevorzugt besteht das Führungselement aus Kunststoff. Bevorzugt ist das Führungselement an der Spitze weich und flexibel. Bevorzugt ist die Drainageleitung ein Schlauch. Bevorzugt besitzt die Drainageleitung am distalen Ende seitliche Öffnungen. Bevorzugt besitzt die Drainageleitung am distalen Ende seitliche Öffnungen über die gesamte oder inkomplette Länge des Fluidsammelmittels. Bevorzugt ist eine zusätzliche Drainageleitung in den Schwamm eingeführt. Bevorzugt ist die zusätzliche Drainageleitung in dem Fluidmittel verschieblich. Bevorzugt ist die zusätzliche Drainageleitung in dem Fluidmittel fest befestigt. Bevorzugt ist die zusätzliche Drainageleitung an der distalen Spitze abgerundet. Bevorzugt beinhaltet auch die zusätzliche Drainageleitung ein Führungselement. Bevorzugt verjüngt sich das Fluidsammelmittel am distalen Ende konisch. Bevorzugt verjüngt sich das Fluidsammelmittel am proximalen Ende konisch. Bevorzugt sind mehrere Fluidsammelmittel auf einem Drainageschlauch befestigt. Bevorzugt ist die Schwammdrainage zur intraluminalen und intracavitären Vakuumschwammtherapie im gesamten Magendarmtrakt, zur Drainage von Körperhöhlen, Hohlorganen, Gewebeabszessen, Intestinallumina, zur Platzierung über natürliche oder künstliche Körperöffnungen mit gleichzeitiger Möglichkeit der zusätzlichen schwammfernen Dekompression, Spülung oder Ernährung bei intraluminaler Schwammplatzierung ausgestaltet.

Durch die Einlage einer Drainage, die mit einem Polyurethanschwamm verbunden ist, lassen sich bekanntermaßen innere Wunden bei postoperativen Anastomoseninsuffizienzen am Enddarm behandeln. Die Drainage wird hierzu unter einem dauerhaften Sog gesetzt. Wundsekret kann nach innen abgeleitet werden, die Wunden reinigen sich und heilen.

An der Speiseröhre konnten Defekte durch Perforationen oder postoperativen Anastomoseninsuffizienzen durch die intraluminale Einlage einer Polyurethanschwammdrainage behandelt werden. Eine Schwammdrainage wurde dazu über dem Defekt in das Intestinallumen eingelegt. Wenn die Drainage dann unter Sog gesetzt wird, verschließt sich das Darmlumen, der Defekt kann verkleben und heilen. Ebenso konnte auch am Zwölffingerdarm die Wirksamkeit der intraluminalen Therapie gezeigt werden. Mit der Vakuumschwammtherapie ist eine endoskopische Behandlung dieser Verletzungen möglich. Durch das bislang verfügbare Einführsystem lassen sich nur körperöffnungsnahe Anastomoseninsuffizienzen im Enddarm versorgen.

Die mit der Erfindung erzielbaren Vorteile und neuen Behandlungsmöglichkeiten sind zahlreich. Bei der Erfindung handelt es sich um eine Schwammdrainage, welche unter endoskopischer, radiologischer oder operativer Sicht z. B. in einem Darmlumen platziert wird. Die Platzierung erfolgt über ein Überschieben der Drainage über einem zuvor endoskopisch eingelegten Führungsdraht. Endoskopisch wird ein Führungsdraht intraluminal eingelegt und anschließend unter endoskopischer, radiologischer oder operativer Kontrolle die Schwammdrainage entlang des Führungsdrahtes exakt zum gewünschten Platzierungsort geschoben. Die Erfindung kann intraluminal im gesamten Magendarmtrakt eingelegt werden. Durch die Einlage eines zusätzlichen Drainageschlauches in den Schwamm, eröffnen sich neue Behandlungsmöglichkeiten. Wenn dieser zusätzliche Drainageschlauch in dem Schwamm verschieblich konstruiert wird, kann er nach der Positionierung der Schwammdrainage vorgeschoben oder auch entfernt werden, Wenn z. B. eine Behandlung einer Speiseröhrenverletzung vorgenommen wird, kann der zusätzliche Drainageschlauch in den Magen vorgeschoben werden und hierüber eine enterale Ernährung oder eine Dekompression zur Entlastung des Magens erfolgen. Diese Verfahren sind auch dann möglich, wenn der Schwamm unter Unterdruck gesetzt wird und das Speiseröhrenlumen verschließt. Durch den Unterdruck saugt sich zum einen der Schwamm im Darmlumen fest, so dass er sich nicht verschiebt, zum anderen saugt er auch die zusätzliche Drainage fest und fixiert diese im Schwammdurchtritt. Wenn eine Behandlung von anal vorgenommen wird und ein Verschluss des Enddarmes, z. B. zur Sicherung einer Anastomose oder vorübergehenden Ausschaltung eines Darmabschnittes mit der Erfindung vorgenommen wird, kann über diesen zusätzlichen (oral des endoskopischen passageren Darmverschlusses gelegenen) Schlauch eine Entlastung von Darmgasen oder Stuhl stattfinden und der Dickdarm dekomprimiert werden. Es ist auch eine Spülbehandlung möglich. Es ist auch die intraoperative intraluminale Platzierung im Dünndarm möglich. Beispielsweise kann zur Behandlung einer Dünndarmperforation der Schwamm in Höhe eines Perforationsdefektes oder einer Anastomose eingelegt werden. Durch das Vakuum wird das Darmlumen passager verschlossen, eine Abheilung eines Defektes ist dann möglich. Gleichzeitig ist durch eine zusätzliche Sonde, deren distales Ende aboral des Schwammes zu liegen kommt, die enterale Ernährung möglich.

Bevorzugt bezieht sich die Erfindung auf ein Drainagesystem zum Absaugen von Körperflüssigkeiten, Wundsekreten, Gasen aus Körperhöhlen, Hohlorganen, Gewebeabszessen, Intestinallumina, zum passageren endoskopischen Verschluss von Intestinallumina sowohl über natürliche als auch künstliche Körperöffnungen, wobei das Drainagesystem umfasst: Drainageschlauch, Fluidsammelmiltel und Overtubehülse für ein Endoskop.

Bevorzugt besitzt das Fluidsammelmittel eine Röntgenmarkierung. Bevorzugt ist das Fluidsammelmittel röhrenförmig mit einem zentralen Lumen konstruiert ist. Bevorzugt umfasst das Fluidsammelmittel eine elastisch komprimierbare Struktur mit Fluidkanälen. Bevorzugt umfasst das Fluidsammelmittel einen offenporigen Polyurethanschwamm. Bevorzugt sind eine oder mehrere Drainageleitungen in dem Fluidsammelmittel befestigt. Bevorzugt sind die Drainageleitungen Schläuche. Bevorzugt besitzt die Drainageleitung am distalen Ende seitliche Drainageöffnungen. Bevorzugt ist die Drainageleitung am distalen Ende über die gesamte Länge, die sich in dem Fluidmittel befindet, mit seitlichen Drainageöffnungen versehen. Ferner bezieht sich die Erfindung auf einen Overtube für ein vorstehend erläutertes Endoskop, wobei der Overtube aus einem Kunststoffschlauch besteht.

Zur Behandlung von sekundär heilenden Wunden werden im Stand der Technik offenporige Polyurethanschwämme in Wunden eingelegt, mit einer Folie nach außen verschlossen, mit einem Drainageschlauch verbunden und mit Hilfe eines Vakuumpumpensystems unter Sog gesetzt. Die Wunde kollabiert unter dem Unterdruck, das Wundsekret wird von der Wunde kontinuierlich oder intermittierend abgesaugt. Die Wunde kann sich rasch reinigen, verkleinern und Granulationsgewebe ausbilden. Dass dieses Therapiekonzept auch an inneren Wunden effektiv ist, konnte an Wunden in Folge von Nahtrupturen nach Enddarmoperationen nachgewiesen werden. Hierzu wird eine Polyurethanschwammdrainge durch die Nahtruptur hindurch in eine dahinterliegende Wundhöhle eingebracht.

Ebenso konnte gezeigt werden, dass die Vakuumschwammtherapie auch in tieferen nur mit einem Endoskop zu erreichenden inneren Wunden z.B. als Folge von Operationen oder Verletzungen der Speiseröhre oder des Zwölffingerdarms effektiv ist. Dazu wird eine Vakuumschwammdrainage unter endoskopischer Sicht entweder über einen Defekt in eine Wundhöhle eingebracht oder die Schwammdrainage wird im Darmlumen über dem Defekt eingelegt. Es konnte auf diese Art und Weise ein kompletter passagerer Verschluss des Darmlumens erzielt werden. Diesen künstlich herbeigeführten Darmverschluss durch die Vakuumschwammdrainage kann man therapeutisch nutzen. Es wird eine Kontamination der inneren Wunde durch die Verdauungssekrete verhindert, gleichzeitig werden die Wundsekrete abgesaugt, die Wunde kann sich reinigen und Defekte gleichzeitig durch das Ansaugen mit dem Vakuumsog am Schwamm sich verschließen. Die endoskopische Platzierung einer Schwammdrainage kann technisch schwierig sein. Mit dem derzeit verfügbaren System lassen sich nur körpernahe innere Wunden behandeln.

Durch die Erfindung wird die endoskopische Platzierung einer Vakuumschwammdrainge technisch einfach. Die neuen medizinischen Behandlungsmöglichkeiten, die sich durch die Drainage von inneren Wunden oder die Herbeiführung eines Verschlusses des Intestinalumens herbeiführen lassen, sind vielfältig.

Bei der Erfindung handelt es sich um einen offenporigen röhrenförmig konstruierten Polyurethanschwammkörper, variabler Länge. Dieser Schwamm wird mit dem Distalende eines Drainageschlauches, welcher am distalen Ende Perforationsöffnungen besitzt, fest verbunden. Der Schwammkörper wird auf einem, durch einen Overtube geführtes, Endoskop aufgesetzt, er ist auf diesem verschieblich. Bei dem Overtube handelt es sich um eine auf dem Endoskop verschiebliche schlauchartige Hülse. Der Overtube liegt proximal der röhrenförmigen Schwammdrainage. Zum einen verhindert er, dass der Schwamm auf dem Endoskop nach proximal rutschen kann. Zum anderen wird der Overtube als Pusher dazu benutzt, die Schwammdrainage auf dem Endoskop gleitend, nach distal zu führen und vom Endoskop abzuschieben und so freizusetzen. Es kann auf diese Weise eine Schwammdrainage sowohl in eine Wund- oder Körperhöhle oder z. B. intraluminal in einem Darmlumen oder Hohlorgan eingebracht werden. Sobald die Schwammdrainage freigesetzt ist, wird ein Unterdruck an diese angelegt, so dass sie sich festsaugt. Dann kann das Endoskop entfernt werden.

Vor der Schwammplatzierung ist es im selben Arbeitsschritt möglich über den Arbeitskanals eines Endoskops eine Sonde vorzuschieben. Bei einem endoskopischen Einlegen einer Schwammdrainage in der Speiseröhre, kann über diese z. B. bis in Magen oder Dünndarm eingebrachte Sonde, eine enterale Ernährung oder die Verabreichung von oralen Medikamenten erfolgen.

Bevorzugt bezieht sich die Erfindung auf ein Drainagesystem zum Absaugen von Körperflüssigkeiten, Wundsekreten, Gasen aus Körperhöhlen, Hohlorganen, Gewebeabszessen, Intestinallumina, mit Drainageleitungen, Fluidsammelmittel und Verbindungselementen für die Drainageleitungen. Bevorzugt umfasst das Fluidsammelmittel eine elastisch komprimierbare Struktur mit Fluidkanälen. Bevorzugt umfasst das Fluidsammelmittel einen offenporigen Polyurethanschwamm. Bevorzugt sind eine oder mehrere Drainageleitungen in dem Fluidsammelmittel befestigt. Bevorzugt sind die Drainageleitungen Schläuche. Bevorzugt besitzt die Drainageleitung am distalen Ende seitliche Drainageöffnungen. Bevorzugt ist die Drainageleitung am distalen Ende über die gesamte Länge, die sich In dem Fluidmittel befindet, mit seitlichen Drainageöffnungen versehen. Bevorzugt kann das Fluidsammelmittel in kleinere Drainagesystemeinheiten getrennt werden. Bevorzugt ist das Fluidsammelmittel zur Teilungsmöglichkeit in kleinere Drainageeinheiten mit Perforationsschlitzen versehen. Bevorzugt können die Drainageschläuche mit Verbindungselementen miteinander verbunden werden.

Zur Ableitung von Wundsekreten, Körperflüssigkeiten, Vereiterungen werden üblicherweise nach Operationen Drainageschläuche eingelegt. Diese sollen Sekrete über seitliche Öffnungen im Drainageverlauf im Sinne einer Schwerkraftdrainage und Überlaufdrainage ableiten. Drainagen können als Schlauchdrainage oder auch als flächige Drainagen konstruiert sein. Bei letzteren gelingt eine Drainage auch über eine Kapillarwirkung. Es werden auch Drainageschläuche eingelegt, die an einen Unterdruck angeschlossen werden. Die Wunddrainage entfalten ihre Wirkung meist aber nur unmittelbar nach einer Operation, da es unter anderem durch Fibrinausfällung, Koagulation von Blut und dem Anliegen von Gewebe zu einer schnellen Verstopfung der Drainageöffnungen kommt.

In der Behandlung von offenen sekundär heilenden Weichteilwunden hat sich in den letzten Jahren die Vakuumschwammtherapie etablieren können. Hierbei wird ein Polyurethanschwamm, der in eine Wunde eingelegt und mit einem Folienverband verschlossen wird, als Filter zur dauerhaften längerfristigen über Tagen durchzuführenden Wunddrainage genutzt. Die Wunden reinigen sich meist zügig und es gelingt insbesondere auch problematischen Wunden zur Abheilung zu bringen. Auch zur Behandlung an inneren Wunden, die infolge von Anastomoseninsuffizienzen oder Perforationen am Darm entstehen, wurde die endoskopische Vakuumschwammtherapie mit Erfolg eingesetzt. Zur Behandlung von septischen Wunden in der Bauchhöhle bei z. B. bei einer Bauchfellentzündung werden ebenfalls Polyurethanschwämme zur Sekretableitung eingesetzt. Diese z. T. großflächigen Wundverbände werden mit einer Folie in Form eines Okklusionsverbandes am offenen Abdomen mit ein oder zwei Drainageansätzen unter einen Unterdruck gesetzt.

Die Erfindung birgt mehrere verbesserte und erweiterte Einsatzmöglichkeiten der Vakuumschwammtherapie. Bei der Erfindung handelt es sich um einen mit Drainageschläuchen versehenen offenporigen Schwamm, welcher sich in kleinere Untereinheiten teilen lässt. Die Drainageschläuche sind über ein Verbindungselement miteinander verbunden, Die Erfindung ermöglicht z. B. bei Abdominaloperationen eine gezielte Platzierung von Vakuumschwammdrainagen exakt am eigentlichen septischen intraabdominellen Fokus. Zudem ist die Drainage von den "tiefsten Punkten", an denen sich Wundwasser bzw. Wundsekret in der Bauchhöhle sammeln kann, möglich. Es ist auch die Platzierung in Hohlorgane wie z. B. einem Darmlumen möglich. Hierdurch ergeben sich neue Behandlungsmöglichkeiten.

Die Erfindung kann operativ in Form von großflächigen Schwammpaketen in die Bauchhöhle eingebracht werden, es ist aber auch eine Teilung in mehrere Untereinheiten möglich. Hierzu ist der Schwamm an Untereinheiten vorperforiert, um die Untereinheiten durch einfaches Auseinanderziehen der Einheiten bewerkstelligen zu können. Über Verbindungselemente werden die einzelnen Drainageschläuche innerhalb oder außerhalb der Bauchhöhle miteinander verbunden, so dass nur ein einzelner Schlauch an einen Unterdruck angeschlossen werden muss. An alle Untereinheiten kann so ein Unterdruck angelegt werden. Es ist möglich die Drainageschläuche zu spülen und sie als Spülschläuche zu verwenden, um eine sogenannte Spül-Saugbehandlung durchzuführen.

Dadurch, dass über die gesamte Länge eines Schwammelementes das perforierte Ende des Drainageschlauches im Schwamm befestigt ist, ist ein Vorteil der Erfindung, dass es nach der Anlage des Unterdrucks zum Zusammenziehen des Schwammes in der Breite aber nur unwesentlich zu einer Längenverkürzung kommt. Auf diese Weise bleibt der die ursprüngliche Platzierung des Schwammes gesichert.

Die Erfindung ist in den Fällen einzusetzen, in denen wiederholte kurzfriste operative Eingriffe aufgrund von septischen Bedingungen zu erwarten sind (sogenannte Relaparotomien). Die Erfindung muss in der Regel unter konventionellen operativen Bedingungen (d.h. Eröffnung der Körperhöhle über einen Schnitt) eingebracht und entfernt werden. Ihr Einsatz wird zunächst auf Fälle von schwersten septischen Krankheitsbildern beschränkt bleiben, da ein Risiko eine iatrogene Verletzung von Organen durch die direkte Sogwirkung am Schwamm ist. Die Vakuumschwammdrainage wird nach Eröffnung des Abdomen gezielt platziert und anschließend die Abdominalhöhle wieder mit einer Naht verschlossen, so dass durch den wieder verschlossenen Raum ein Sog aufgebaut werden kann. Es ist auch die Verwendung der Erfindung in Kombination mit einem luftdichten Folienverband als Bauchhöhlenverschluss möglich. Es ist auch die minimal invasive z. B. laparoskopische Platzierung der Erfindung möglich. Die Erfindung ist auch in anderen Körperhöhlen, Thoraxhöhle, Infekthöhlen, Organhöhlen, Gewebeabzeßen, Intestianllumina, etc. verwendbar.

Ausführungsformen, die Kombinationen der vorstehend erläuterten Merkmale aufweisen, sind besonders bevorzugt,

Bevorzugt ist der Kanal parallel zu einer Längsachse des Fluidsammelelementes angeordnet. Bevorzugt ist das Fluidsammelelement, insbesondere kreis-, zylindrisch ausgebildet und der Kanal ist parallel zur Höhe des zylindrischen Fluidsammelelements angeordnet. Bevorzugt ist die Längsachse des Fluidelementes diejenige, welche bei bestimmungsgemäßem Gebrauch des Fluidelementes im Wesentlichen parallel zu der Innenwand desjenigen Bereiches des Körpers orientiert ist, im welchem das Fluidsammelelement angeordnet ist. Ferner ist bevorzugt, dass der Kanal entlang einer Symmetrieachse des Fluidsammelelementes angeordnet ist. Bevorzugt ist das Fluidsammelelement röhrenförmig ausgebildet, wobei der Kanal der innere Hohlraum in dem röhrenförmigen Fluidsammelelement ist. Bevorzugt weist der Kanal eine erste Öffnung als Eingang für das Führungsselement und/oder das Versorgungselement und eine zweite Öffnung als Ausgang für das Führungsselement und/oder das Versorgungselement auf. Bevorzugt sind die erste und die zweite Öffnung auf sich im Wesentlichen gegenüberliegenden Oberflächen des Fluidsammelelementes angeordnet. Vorteilhaft wird erreicht, dass der Kanal zum Führen des Führungsselements, insbesondere eines Führungsdrahts, und/oder des Versorgungselements, insbesondere einer Sondeneinheit und/oder einem Endoskop, durch das Fluidsammelelement genutzt wird, indem er eine Verbindung zwischen dem proximalen und distalen Ende des Fluidsammelelement bildet. Bei der symmetrischen Anordnung des Kanals im Fluidsammelelement werden die Vorteile erreicht, dass das Fluidsammelelement besonders gleichmäßig um einen beispielsweise in dem Kanal angeordneten Drainageschlauch kollabiert, falls dieser einen Unterdruck anlegt, dass Medikamente, die über ein in dem Kanal angeordnetes Versorgungselement dem Fluidsammelelement zugeführt werden, besonders gleichmäßig in dem Fluidsammelelement und auf dessen Oberfläche verteilt werden können, und dass die Führung entlang des Führungselementes besonders einfach ist, da das Fluidsammelelement mit identischem Radius um das Führungselement angeordnet ist. Eine außerhalb der Symmetrieachse aber zu dieser parallele Anordnung des Kanals bietet die Vorteile, dass ein bestimmter Bereich des Körpers, im welchem das Fluidsammelelement bei bestimmungsgemäßem Gebrauch angeordnet ist und welcher dem Kanal näher gelegen ist als der gegenüberliegende Bereich im Körper, den Einflüssen von Unterdruck, Medikament-Zuführung etc. stärker ausgesetzt werden kann als der gegenüberliegende Bereich. Die parallele, insbesondere gerade, Anordnung des Kanals bietet den Vorteil, dass die Reibungskraft beim Führen des Fluidsammelelementes entlang des Führungselementes und beim Einführen des Versorgungselementes in das Fluidsammelelement vorteilhaft reduziert werden kann. Das Verschieben des Fluidsammelelementes gegenüber dem Führungselement und/oder Versorgungselement wird so vorteilhaft vereinfacht.

Bevorzugt weist das Fluidsammelelement einen Längsschlitz entlang der Längsachse des Fluidsammelelementes auf, der sich zwischen der Oberfläche des Fluidsammelelementes und dem Kanal erstreckt. Bevorzugt erstreckt sich der Längsschlitz zwischen den distalen und proximalen Enden und über die gesamte Länge des Fluidsammelelementes. Bevorzugt ist der Längsschlitz in gleicher Weise wie der Längsschlitz des Overtubes ausgebildet. Bevorzugt erstreckt sich der Längsschlitz in radialer Richtung des Fluidsammelelements zwischen dem Kanal und der dem Kanal am nächsten gelegenen Oberfläche des Fluidsammelelementes. Ein längsgeschlitztes Fluidsammelelement bietet den Vorteil, dass es jederzeit auf ein Endoskop, Führungselement, Versorgungselement etc. aufgesetzt und in den Körper eingeführt werden kann, ohne das Endoskop oder dergleichen entfernen zu müssen. Auch kann vorteilhaft erreicht werden, dass ein bereits in Benutzung befindliches Fluidsammelelement gegen ein frisches Fluidsammelelement ausgetauscht werden kann, ohne dass das Endoskop oder dergleichen während dieses Austauschs entfernt werden muss. Weitere Vorteile können in analoger Weise erreicht werden, wie sie für den längsgeschlitzten Overtube beschrieben sind. Bevorzugt wird der Längsschlitz im Fluidsammelmittel und/oder Overtube nach dem seitlichen Plazieren auf dem Führungs/Versorgungselement, insbesondere Endoskop oder Sonde, verschlossen, insbesondere mittels Naht, Klebung oder dergleichen. Verschiedene Verschlussmechanismen für das Fluidsammelmittel und/oder dem Overtube sind bevorzugt. Die Verschlussmechanism sind ausgestaltet, um den Längschlitz vollständig oder nur teilweise (beispielsweise nur an der Spitze) zu verschließen, um ein Führen an dem Führungselement, beispielsweise Endoskop, zu vereinfachen. Ein vollständiger Verschluss ist beispielsweise durch eine Art Reissverschluss bevorzugt. Vorteilhaft kann so die Führung in körperöffnungsferne Bereiche und über Abknickungen erreicht werden.

Bevorzugt weist das Fluidsammelelement eine Röntgenmarkierung auf, die ausgestaltet ist, um bei einer Röntgenaufnahme detektiert zu werden. Bevorzugt ist die Röntgenmarkierung auf der Oberfläche des Fluidsammelelementes angeordnet. Bevorzugt ist die Röntgenmarkierung als Kreis um ein rotationssymmetrisch ausgestaltetes Fluidsammelelements angeordnet. Bevorzugt ist die Röntgenmarkierung im Inneren des Fluidsammelelements angeordnet. Die Anordnung einer Röntgenmarkierung an dem Fluidsammelelement weist den Vorteil auf, dass dessen Positionierung und auch die komplette Entfernung kontrolliert erfolgen und bei bestimmungsgemäßem Gebrauch der Vakuumschwammeinheit überwacht werden kann.

Bevorzugt ist das Fluidkommunikationselement durch eine Fluidkommunikationselementöffnung in das Fluidsammelelement eingeführt und erstreckt sich entlang der vollständigen Länge des Fluidsammelelementes. Bevorzugt erstreckt sich das Fluidkommunikationselement entlang der vollständigen Längs- und/oder Symmetrieachse. Vorteilhaft wird erreicht, dass der Einfluss (Anlegen eines Unterdrucks/Überdrucks, Einführen eines Medikamentes etc.) des Fluidkommunikationselements über die gesamte Länge des Fluidsammelelementes im Wesentlichen identisch ist. Ferner wird erreicht, dass sich die Positionierung des Fluidsammelelementes, insbesondere des distalen Endes des Fluidsammelelements, im Wesentlichen nicht ändert, wenn das Fluidkommunikationselement einen Unterdruck, Überdruck, Medikament etc. an das Fluidsammelelement anlegt.

Bevorzugt ist das Fluidkommunikationselement in dem Kanal angeordnet. Alternativ ist bevorzugt, dass das Fluidkommunikationselement außerhalb des Kanals angeordnet ist. Bevorzugt ist das Fluidkommunikationselement beabstandet von dem Führungselement und/oder dem Versorgungselement in dem Fluidsammelelement angeordnet. Bevorzugt ist das Führungs- und Versorgungselement in dem Fluidkommunikationselement, insbesondere dem Drainageschlauch angeordnet. In einer bevorzugten Ausführungsform ist eine Ernährungssonde in dem Drainageschlauch angeordnet und ausgestaltet, um vorgeschoben zu werden, wodurch auch noch nach der Platzierung des Fluidsammelelementes eine Sonde zur Ernährung durch den Kanal eingelegt, oder von vornherein so platziert werden kann, wodurch vorteilhaft Platz gespart wird. Je nach Art des zu versorgenden Bereiches des Körpers kann eine besonders kompakte Ausgestaltung der Vakuumschwammeinheit erreicht werden. Je nach anatomischer Ausgestaltung des zu versorgenden Bereiches und des Weges innerhalb des Körpers zu diesem Bereich kann die Anordnung von Fluidkommunikationselement zu Führungselement und/oder Versorgungselements vorteilhaft gewählt werden.

Bevorzugt ist das Fluidkommunikationselement ausgestaltet, um mit einer, insbesondere elektrischen, Vakuumpumpe fluidleitend verbunden zu werden. Die Vakuumpumpe ist bevorzugt ausgestaltet, um einen Unterdruck und/oder einen Überdruck an das Fluidkommunikationselement anzulegen. Das Fluidkommunikationselement weist einen Adapter auf, um es direkt oder indirekt mit der Vakuumpumpe zu verbinden. Vorteilhaft wird erreicht, dass das Fluidsammelelement besonders effektiv und präzise mit einem Unterdruck beaufschlagt werden kann. Der Unterdruck ist kontinuierlich oder intermittierend. Da bei der endoskopischen Platzierung der Schwamm über den Sog fixiert wird, existiert immer ein bestimmter Unterdruck, sonst kann der Schwamm dislozieren. Es ist auch bevorzugt, dass der Unterdruck intermittierend angelegt wird, zwischen negativen Drücken schwankend (z. B. zwischen 80 und 125 mmHG). Das hat den Vorteil, dass der durch den Schwamm erzeugte Gewebedruck eine geringere mögliche Druckschädigung auf das Gewebe ausüben kann als bei erhaltener Fixierung durch den Negativdruck.

Bevorzugt ist das Fluidkommunikationselement fest mit dem Fluidsammelelement verbunden ist. Bevorzugt ist das Fluidkommunikationselement mit dem Fluidsammelelement verklebt, verschweißt, vernäht oder an diesem andersartig mechanisch fixiert. Vorteilhaft wird eine besonders zuverlässige Fluidleitung zwischen Fluidkommunikationselement und Fluidsammelelement erreicht. Ferner wird vorteilhaft erreicht, dass das Fluidsammelelement an dem Fluidkommunikationselement aus dem Körper gezogen werden kann.

Bevorzugt weist das Fluidkommunikationselement in demjenigen Bereich, welcher in dem Fluidsammelelement angeordnet ist, eine Vielzahl von Öffnungen auf. Bevorzugt sind die Öffnungen Perforationen. Bevorzugt sind die Öffnungen symmetrisch in der Oberfläche des Fluidkommunikationselementes angeordnet. Bevorzugt sind die Öffnungen nur auf einer Seite der Oberfläche des Fluidkommunikationselementes angeordnet, um ein asymmetrisches Kollabieren des Fluidsammelelements um das Fluidkommunikationselement zu erreichen. Mittels der Vielzahl von Öffnungen wird vorteilhaft eine im Wesentlichen gleichmäßige Verteilung des Einflusses des Fluidkommunikationselements auf den den Öffnungen gegenüberliegenden Bereich des Fluidsammelelements erreicht.

Bevorzugt weist die Vakuumschwammeinheit ein zusätzliches Fluidsammelelement auf, welches an dem Fluidkommunikationselement angeordnet ist. Bevorzugt weist das zusätzliche Fluidsammelelement die vorliegend beschriebenen Merkmale des Fluidsammelelementes auf. Bevorzugt sind das Fluidsammelelement und das zusätzliche Fluidsammelelement anliegend aneinander an dem Fluidkommunikationselement angeordnet. Vorteilhaft kann dadurch ein längerer Bereich des Körpers versorgt werden. Alternativ ist bevorzugt, dass das Fluidsammelelement und das zusätzliche Fluidsammelelement nicht anliegend aneinander (d.h. beabstanden voneinander) an dem Fluidkommunikationselement angeordnet sind. Vorteilhaft kann mit diesen Ausführungsformen erreicht werden, dass bei unterschiedlicher Ausgestaltung des zusätzlichen Fluidsammelelements und des Fluidelementes ein oder mehrere Bereiche im Körper unterschiedlich versorgt werden kann. Bevorzugt wird jedes Fluidsammelmittel einzeln unter einen Unterdruck gesetzt. Es kann auch eine lokale Spülbehandlung und/oder eine Saugbehandlung vorgenommen werden.

Bevorzugt ist das Fluidsammelelement ausgestaltet, um ein Gas und/oder eine Flüssigkeit, welche(s) insbesondere Partikel aufweist(en), von einer äußeren Oberfläche des Fluidsammelelementes zu dem Fluidkommunikationselement (oder in umgekehrter Richtung (für Medikamente, Spülung etc.)) zu leiten. Bevorzugt ist das Fluidkommunikationselement ausgestaltet ist, um ein Gas und/oder eine Flüssigkeit, welche(s) insbesondere Partikel aufweist(en), von dem Fluidsammelelement abzuleiten. Bevorzugt ist das Fluidsammelelement ein offenporiger Polyurethanschwamm. Bevorzugt weist das Fluidsammelelement eine Porengröße von 200µm bis 800 µm, insbesondere bevorzugt von 400 µm bis 600 µm, auf, aber es sind auch andere Porengrößen möglich. Bevorzugt ist das Fluidkommunikationselement ein Drainageschlauch.

In einem weiteren Aspekt bezieht sich die Erfindung auf ein Vakuumschwammsystem mit einer Vakuumschwammeinheit gemäß der Erfindung und einem Führungsselement, insbesondere einem Führungsdraht, und/oder einem Versorgungselement, insbesondere einer Sondeneinheit und/oder einem Endoskop. Die vorstehend erläuterten Ausgestaltungen und Vorteile der Vakuumschwammeinheit gelten in analoger Weise für das Vakuumschwammsystem.

Bevorzugt weist das Vakuumschwammsystem ferner einen Overtube zum Umhüllen der Vakuumschwammeinheit, des Führungsselements und/oder des Versorgungselements aufweist. Der Overtube weist einen kleineren Innendurchmesser als der Außendurchmesser des Fluidsammelelements auf. Der Overtube ist ausgestaltet, um das Fluidsammelelement zusammengedrückt zu führen. Der Overtube wird vorteilhaft verwendet, um das zusammengedrückte Fluidsammelelement entlang des Führungselementes, insbesondere des Endoskops, zu dem zu versorgenden Bereich innerhalb des Körpers zu führen.

Bevorzugt ist der Overtube entlang der Längsachse des Overtubes vollständig geschlitzt. Bevorzugt ist der Overtube zwischen proximalem und distalem Ende geschlitzt. Vorteilhaft wird erreicht, dass der Overtube jederzeit auf ein Führungselement und/oder Versorgungselement aufgesetzt oder von diesem entfernt werden kann.

Bevorzugt weist der Overtube ein erstes Ende, welches konisch ausgestaltet ist, und/oder ein zweites Ende auf, welches trichterförmig ausgestaltet ist. Bevorzugt ist das erste Ende das distale Ende und das zweite Ende das proximale Ende des Overtubes. Vorteilhaft wird erreicht, dass das distale Ende besonders einfach in den Körper eingebracht und bis zu dem zu versorgenden Bereich geführt werden kann. Durch das trichterförmig ausgestattete Ende wird vorteilhaft erreicht, dass das Einführen von Führungselement, Versorgungselement, Vakuumschwammeinheit und dergleichen besonders einfach erfolgen kann.

Bevorzugt weist der Overtube wenigstens teilweise ein elastisch verformbares Material auf. Bevorzugt ist der Overtube aus Kunststoff gefertigt. Bevorzugt weist das Vakuumschwammsystem ferner eine Positionierungshülse und eine Führungshülse auf, wobei die Führungshülse gegenüber der Positionierungshülse verschiebbar ausgestaltet ist. Bevorzugt weisen die Positionierungshülse und/oder die Führungshülse wenigstens teilweise ein elastisch verformbares Material, insbesondere Kunststoff, auf. Vorteilhaft wird mit Overtube, Positionierungshülse und/oder Führungshülse ein besonders einfaches und sicheres Einführen und Positionieren der Vakuumschwammeinheit ermöglicht.

Bevorzugt weist das Vakuumschwammsystem ferner eine, insbesondere elektrische, Vakuumpumpe auf, die mit dem Fluidkommunikationselement fluidleitend verbunden ist und ausgestaltet ist, um einen Unterdruck von bis zu 200 mmHg, bevorzugt 125mmHg, an das Fluidsammelelement anzulegen. Bevorzugt ist die Vakuumpumpe transportabel ausgestaltet, so dass der mit der erfindungsgemäßen Vakuumschwammeinheit versorgte Patient mobil ist.

Bevorzugt ist das Führungsselement im Verhältnis zu seinem Durchmesser lang und im Verhältnis zu seiner Länge dünn ausgestaltet ist. Besonders bevorzugt ist das Führungselement ein Führungsdraht, ein Endoskop, und/oder eine Sondeneinheit. Bevorzugt ist das Führungselement längsgeschlitzt. Das Versorgungselement ist im Verhältnis zu seinem Durchmesser lang und im Verhältnis zu seiner Länge dünn ausgestaltet ist. Das Führungselement und/oder das Versorgungselement sind bevorzugt drahtartig bzw. schnürenartig ausgestaltet. Bevorzugt ist das Versorgungselement insbesondere eine Sondeneinheit, eine Ernährungseinheit, eine Saugeinheit, eine Drainageeinheit, eine Spüleinheit, ein zusätzliches Fluidkommunikationselement, und/oder eine Arbeitseinheit, insbesondere Greif- oder Schneideinheit. Bevorzugt ist das Führungselement ausgestaltet, um das Fluidsammelelement zu dem zu versorgenden Bereich zu führen und zu positionieren. Bevorzugt ist das Versorgungselement ausgestaltet, um den das positionierte Fluidsammelelement umgebenden Bereich zu versorgen, zu untersuchen, zu beobachten, zu bearbeiten etc. Bevorzugt dient das Versorgungselement auch als Führungselement zum Führen und Positionieren des Fluidsammelelements. Vorteilhaft wird erreicht, dass ein gesondertes Führungselement nicht erforderlich ist, um das Fluidsammelelement zu positionieren, so dass die Vakuumschwammeinheit besonders kompakt ausgebildet sein kann und ein einfaches Einführen in den Körper ermöglicht. Bevorzugt weist das Versorgungselement das Führungselement auf. Auch hierdurch kann eine besonders kompakte Ausgestaltung der Vakuumschwammeinheit erreicht werden.

In einem weiteren Aspekt bezieht sich die Erfindung auf ein Verfahren zum Einführen eines erfindungsgemäßen Vakuumschwammsystems in einen menschlichen oder tierischen Körper, mit den Schritten: Anordnen des Führungsselements, insbesondere des Führungsdrahts, in dem menschlichen oder tierischen Körper, Anordnen des Führungselementes in dem Kanal der Vakuumschwammeinheit, und Einführen der Vakuumschwammeinheit entlang des Führungselementes in den menschlichen oder tierischen Körper. Bevorzugt findet der Schritt des Anordnens des Führungselementes in dem Kanal vor, nach oder gleichzeitig zu dem Schritt des Anordnens des Führungselementes in dem Körper statt.

In einem weiteren Aspekt bezieht sich die Erfindung auf ein Verfahren zum Herstellen einer erfindungsgemäßen Vakuumschwammeinheit mit den Schritten: Anordnen eines Fluidkommunikationselements, insbesondere eines Drainageschlauchs, wenigstens teilweise in einem Fluidsammelelement, insbesondere einer Schwammeinheit, Verbinden des Fluidkommunikationselements fluidleitend mit dem Fluidsammelelement, Ausbilden eines Kanal in dem Fluidsammelelement zum Führen eines Führungsselements, insbesondere eines Führungsdrahts, und/oder eines Versorgungselements, insbesondere einer Sondeneinheit und/oder eines Endoskops, durch das Fluidsammelelement. Erfindungsgemäß können die Schritte auch in einer davon abweichenden Reihenfolge durchgeführt werden.

In einem weiteren Aspekt bezieht sich die Erfindung auf eine Verwendung eines fluidsammelnden Materials zur Herstellung einer erfindungsgemäßen Vakuumschwammeinheit zur chirurgischen, endoskopischen, gastroenterologischen oder therapeutischen Behandlung eines menschlichen oder tierischen Körpers.

In einem weiteren Aspekt bezieht sich die Erfindung auf eine Verwendung eines erfindungsgemäßen Vakuumschwammsystems in einem menschlichen oder tierischen Körper, mit den Schritten: Anordnen des Versorgungselementes, insbesondere der Sondeneinheit und/oder des Endoskops, in dem Kanal der Vakuumschwammeinheit, Einführen oder Einlegen der Vakuumschwammeinheit in den menschlichen oder tierischen Körper, und Aktivieren des Versorgungselementes. Bevorzugt findet der Schritt des Anordnens vor, nach oder gleichzeitig zu dem des Einführens oder Einlegens statt.

In einem weiteren Aspekt bezieht sich die Erfindung auf eine Verwendung einer erfindungsgemäßen Vakuumschwammeinheit als Saugeransatz.

Ausführungsformen, die Kombinationen der vorstehend erläuterten Merkmale aufweisen, sind besonders bevorzugt.

Bevorzugte Ausführungsformen der Erfindung werden im Folgenden anhand von Figuren erläutert, von denen
- Figuren 1 bis 7: ein Einführsystem zeigen;
- Figuren 8 bis 15: einen Overtube zeigen;
- Figuren 16 bis 21: einen Schwammsaugeransatz zeigen;
- Figuren 22 bis 26: eine Schwammdrainage zeigen;
- Figuren 27 bis 32: ein Drainagesystem zeigen; und
- Figuren 33 bis 37: ein weiteres Drainagesystem zeigt.

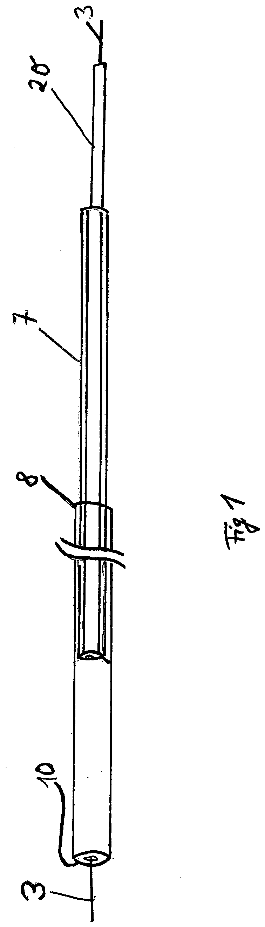

Eine Ausführungsform gemäß der Erfindung ist in Figuren 1 bis 7 gezeigt. Fig. 1 ist eine Darstellung, die die Anordnung des kompletten Einführsystems zeigt. Am distalen Ende der Führungshülse 8 ist der komprimierte Schwamm 10, der mit der Drainage 20 verbunden ist, aufgenommen. Über die Drainage 20 ist eine Positionierungshülse 7 ebenfalls in die Führungshülse eingeschoben, sie ist sowohl gegenüber der Führungshülse als auch der Drainage verschieblich.

In der Drainage liegt der Führungsdraht 3 ein. Dieser Führungsdraht wird zunächst endoskopisch über einen Defekt platziert, dann kann das ganze Einführungssystem über den Draht in die Position geschoben werden. Der Schwamm besitzt eine Röngtenstrahlen-dichte Markierung, so dass unter Röntgenkontrolle die Positionierung vorgenommen und kontrolliert werden kann.

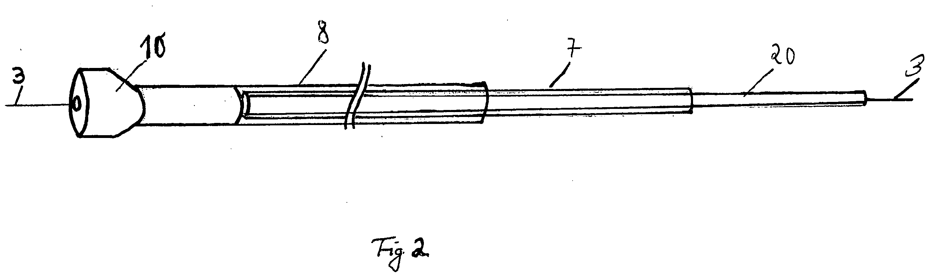

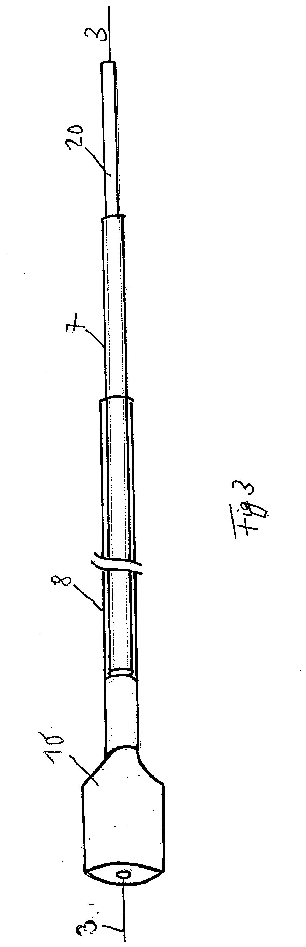

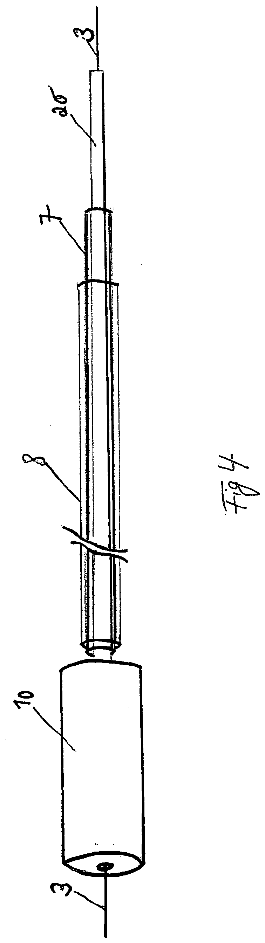

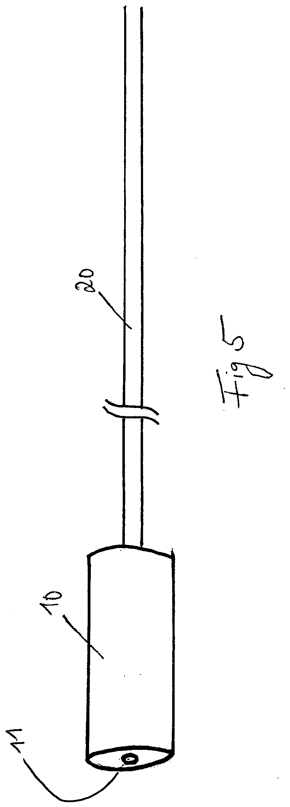

Fig. 2 zeigt das beginnende Freisetzen des Schwammes 10. Die Positionierungshülse 7 und die Führungshülse 8 werden gegeneinander verschoben, so dass der Schwamm aus dem Distalende der Führungshülse austritt. Fig. 3 zeigt ein fortschreitendes Freisetzen des Schwammes. Fig. 4 zeigt eine vollständige Schwammfreisetzung. Fig. 5 zeigt den freigesetzten Schwamm nach Entfernung der Führungshülse, Positionierungshülse und des Führungsdrahtes.

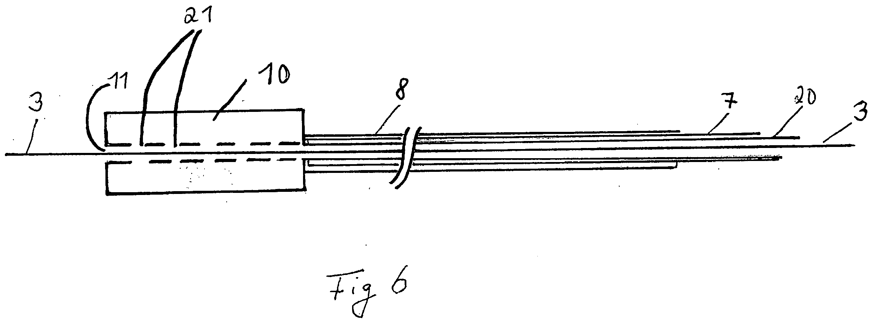

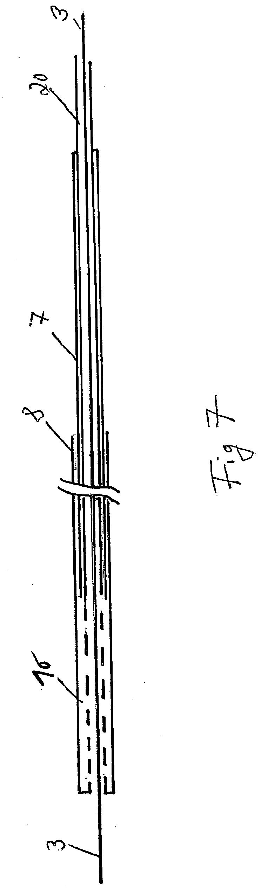

Fig. 6 zeigt einen Querschnitt durch das Einführungssystem mit freigesetztem Schwamm. 21 bezeichnet die Perforationsöffnungen der Drainage im Schwamm. Fig. 7 zeigt einen Querschnitt durch das Einführungssystem mit in dem Distalende der Führungshülse 8 komprimiertem Schwamm 10.

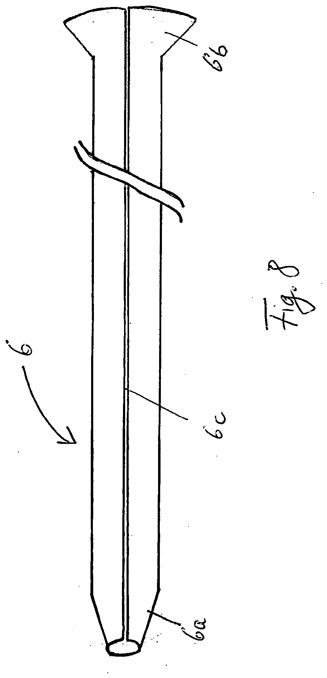

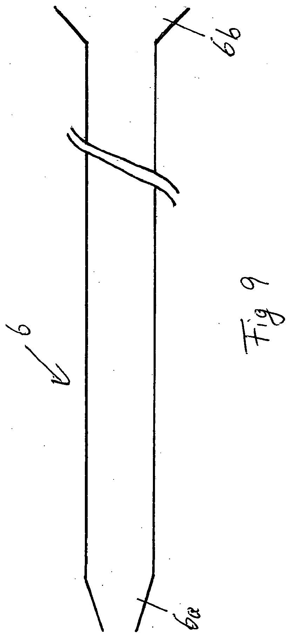

Eine weitere Ausführungsform gemäß der Erfindung ist in Figuren 8 bis 15 gezeigt. Fig. 8 ist eine Darstellung, die die Ausführung des Overtubes zeigt. Am Distalende 6a verjüngt sich der Overtube konisch, um Verletzungen beim Einführen zu vermeiden. Über die ganze Länge ist ein kompletter Schlitz 6c vorhanden. Am proximalen Ende 6b kann der Overtube trichterförmig konstruiert sein, um das Einführen der medizinischen Instrumente zu erleichtern. Fig. 9 ist eine Querschnittdarstellung mit konischem Distalende 6a und trichterförmigem proximalen Ende 6b.

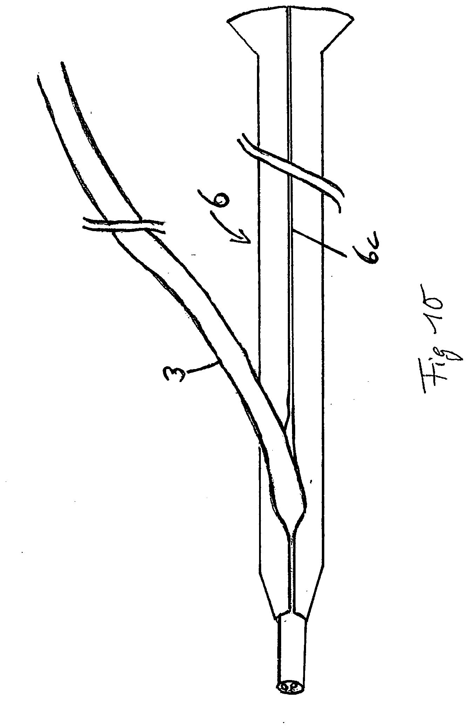

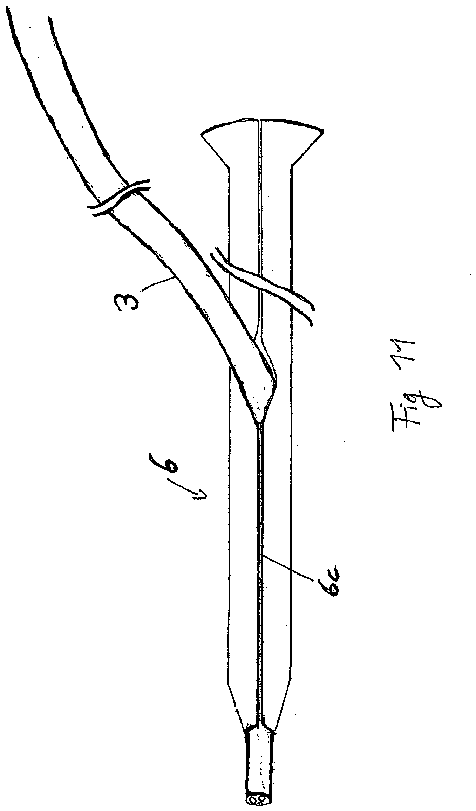

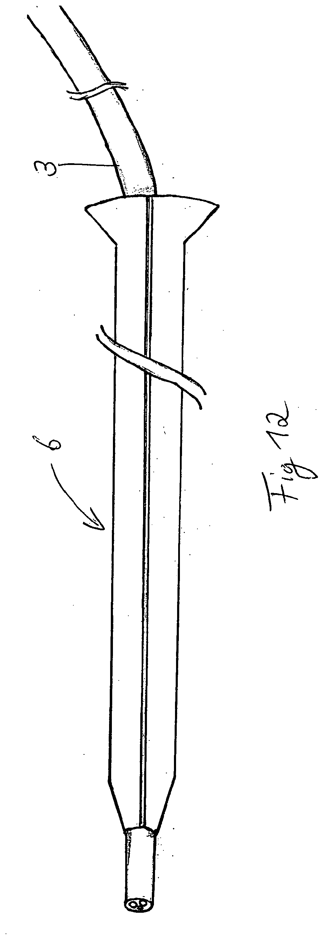

Fig. 10 zeigt, wie ein flexibles Endoskop 3 über den Schlitz 6c am Distalende des Overtubes eingebracht oder entfernt wird. Fig. 11 zeigt, wie ein flexibles Endoskop 3 über den Schlitz 6c in den Overtube eingebracht oder entfernt wird. Fig. 12 zeigt, wie ein das flexible Endoskop 3 vollständig im Overtube eingebracht ist.

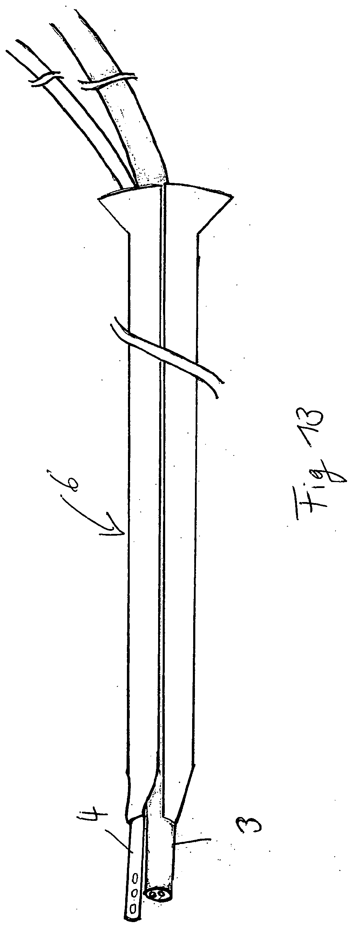

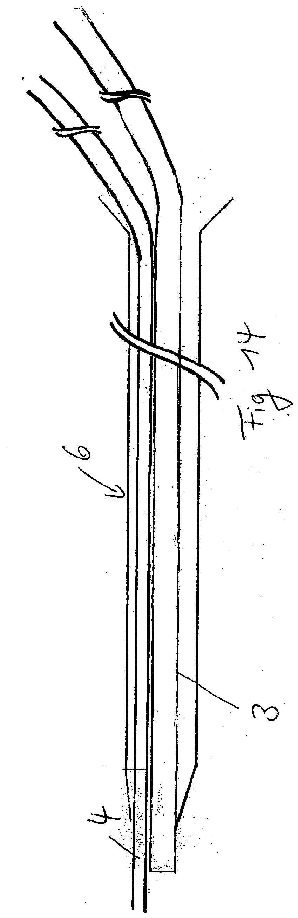

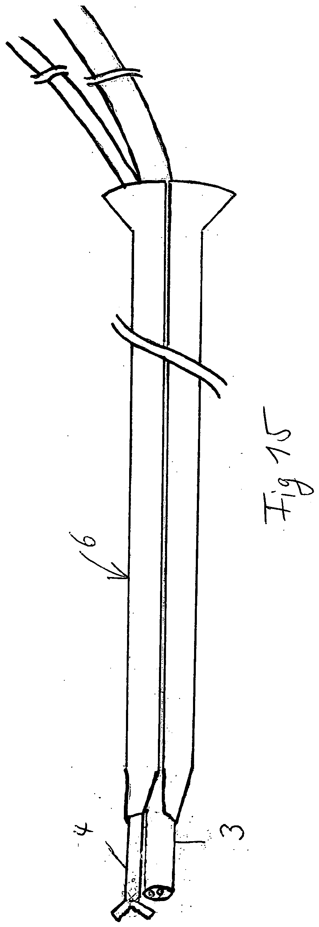

Fig.13 zeigt wie eine Drainage 4 gemeinsam mit einem Endoskop 3 im Overtube eingebracht ist. Fig.14 ist eine Querschnittsdarstellung eines Overtubes mit einem eingebrachten Endoskop 3 und einer Drainage 4. Fig.15 zeigt, wie eine semiflexible Zange 4 als Instrument gemeinsam mit einem Endoskop eingebracht ist.

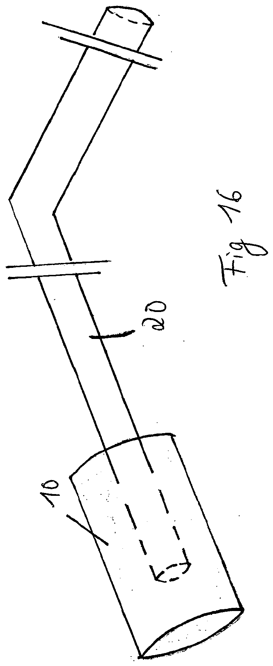

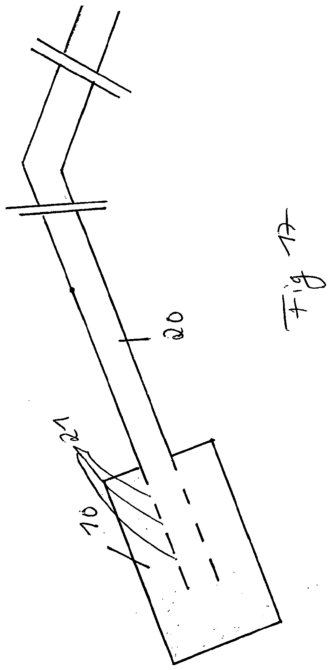

Eine weitere Ausführungsform gemäß der Erfindung ist in Figuren 16 bis 21 gezeigt. Fig. 16 zeigt die Ansicht eines abgeknickten Drainagerohrs 20. mit einem Schwamm 10 am distalen Ende des Drainagerohrs 20. Fig. 17 zeigt die Querschnittdarstellung von Figur 16.

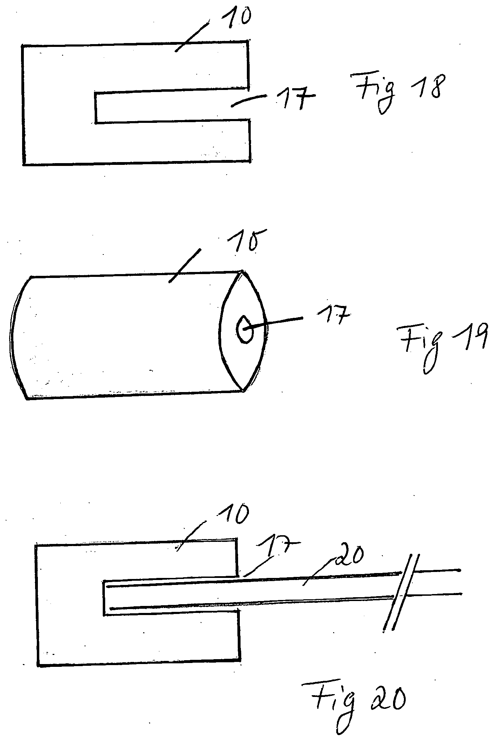

Fig. 18 zeigt eine Querschnittdarstellung eines Schwammes 10 mit zentraler Auslassung 17. Fig. 19 zeigt eine dreidimensionale Ansicht von Figur 18. Fig. 20 zeigt eine Querschnittdarstellung eines Schwammes mit zentraler Auslassung 17, in die ein Drainagerohr 20 eingeführt wurde.

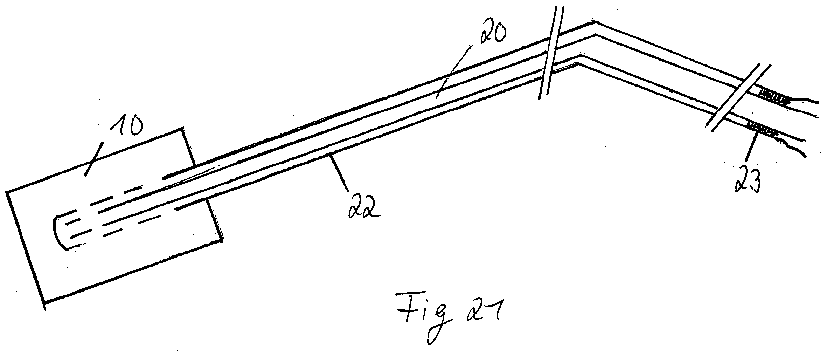

Fig. 21 zeigt einen Querschnitt einer Saugerhülse 22, an deren Distalende ein Schwamm 10 befestigt ist. In die Hülse ist ein zusätzliches Saugerdrainagerohr 20 eingeführt, welches entfernbar ist und über welche die Saugung vorgenommen wird. Saugerhülse 22 und Saugerdrainagerohr schließen bündig 23 miteinander ab.

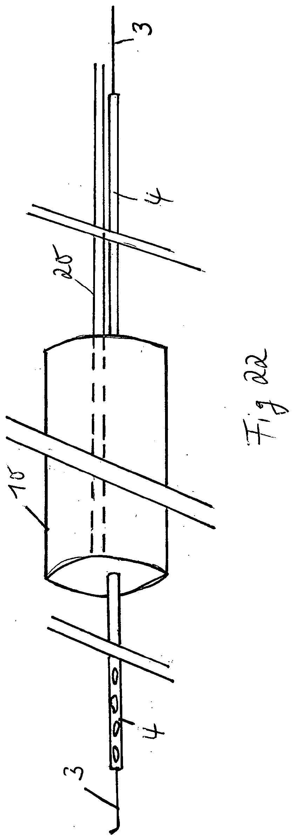

Eine weitere Ausführungsform gemäß der Erfindung ist in Figuren 22 bis 26 gezeigt. Fig. 22 ist eine Darstellung der Schwammdrainage mit Führungsdraht 3, der in einer durch den Schwammkörper 10 geführten Sonde 4 mit seitlichen Perforationsöffnungen am Distalende und der in dem Schwammkörper angeordnet ist. Die in dem Schwammkörper gelegenen Drainage 20 ist über die Länge des Schwammkörpers mit Perforationsöffnungen am Distalende versehen.

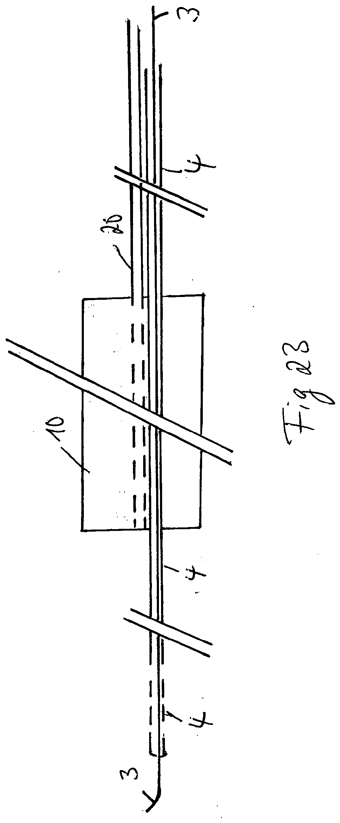

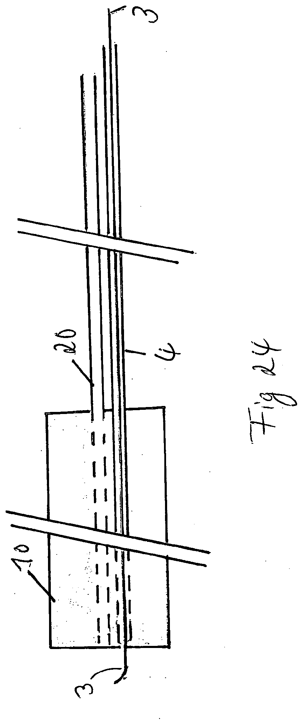

Fig. 23 ist eine Querschnittdarstellung der Anordnung von Figur 22. Fig. 24 ist eine Querschnittdarstellung der Anordnung von Figur 22, wobei die durch den Schwammkörper 10 geführte Sonde 4 verschieblich konstruiert ist und in den Schwammkörper 10 zurückgezogen wurde.

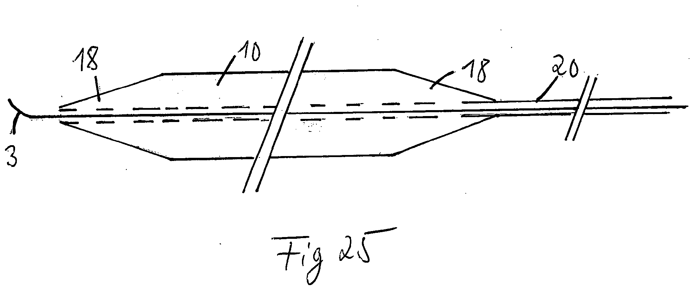

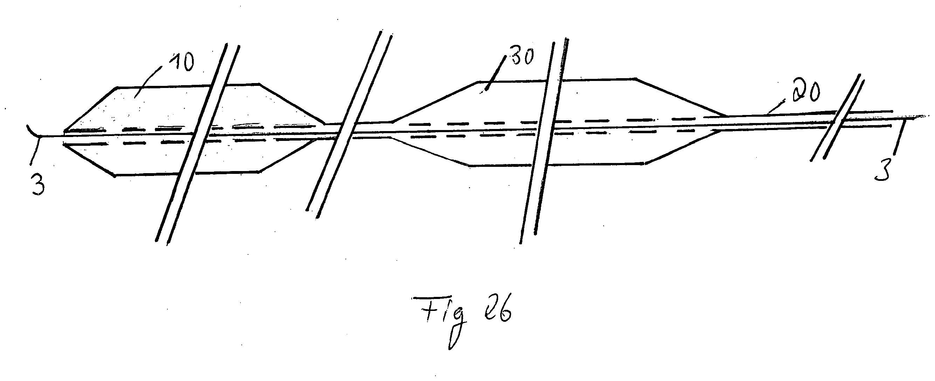

Fig. 25 ist eine Querschnittdarstellung eine Schwammdrainage mit konisch zulaufenden Enden 18 des Schwammkörpers 10, die an einer Drainage 20 mit seitlichen Perforationsöffnungen über die Länge des Schwammkörpers 10 befestigt wurde. Führungsdraht 3 ist in der Drainage 20 angeordnet. Fig. 26 ist eine Querschnittdarstellung einer Schwammdrainage mit zwei konischen Schwammkörpern 10, 30, Führungsdraht 3 und Drainageschlauch 20.

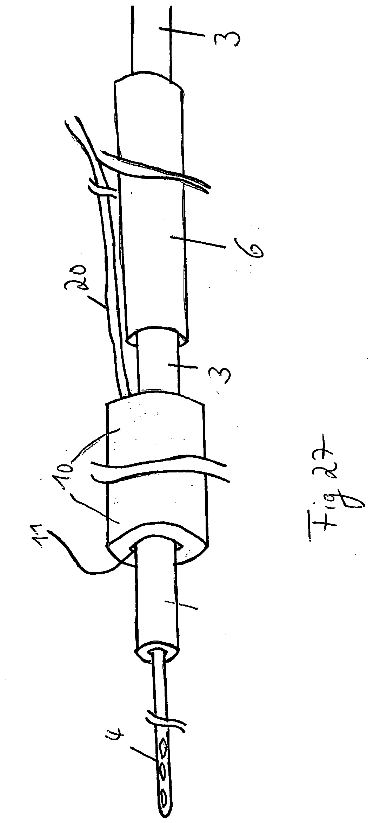

Eine weitere Ausführungsform gemäß der Erfindung ist in Figuren 27 bis 32 gezeigt. Fig. 27 ist eine Darstellung, die die Anordnung des kompletten Drainagesystems (röhrenförmiger Schwamm 10 und Drainageschlauch 20) auf dem distalen Ende eines flexiblen durch einen Overtube 6 geführten Endoskops 3 zeigt. Durch den Arbeitskanal des Endoskops ist eine Ernährungssonde 4 bereits vorgeschoben worden.

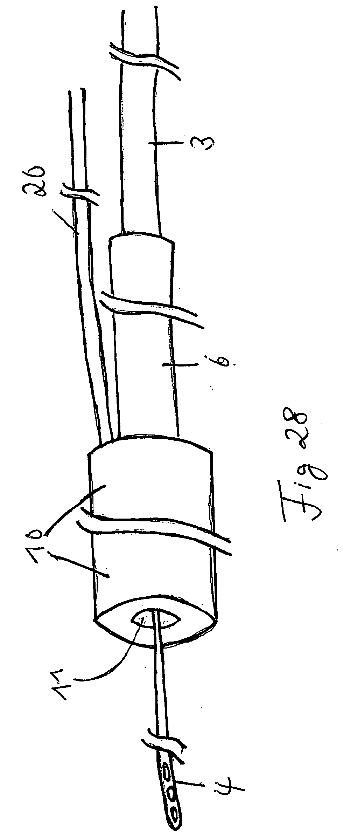

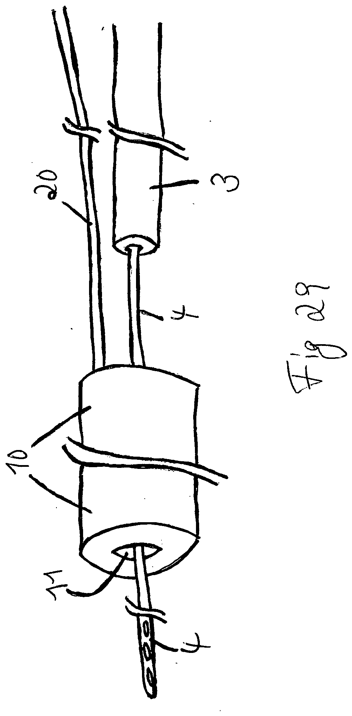

Fig. 28 zeigt die Handhabung der Freisetzung des Schwammes 10. Der Overtube 6 wird auf dem Endoskop 3 nach distal geführt. Der Schwamm rutscht über das distale Ende des Endoskops. Fig. 29 zeigt wie der Schwamm 10 endgültig freigesetzt ist. Zu diesem Zeitpunkt kann über den Drainageschlauch 20 ein Unterdruck angelegt werden. Durch den Unterdruck in einem Darmlumen oder in einer Höhle saugt sich der Schwamm fest, gleichzeitig wird die Ernährungssonde 4 im Schwamm fixiert.

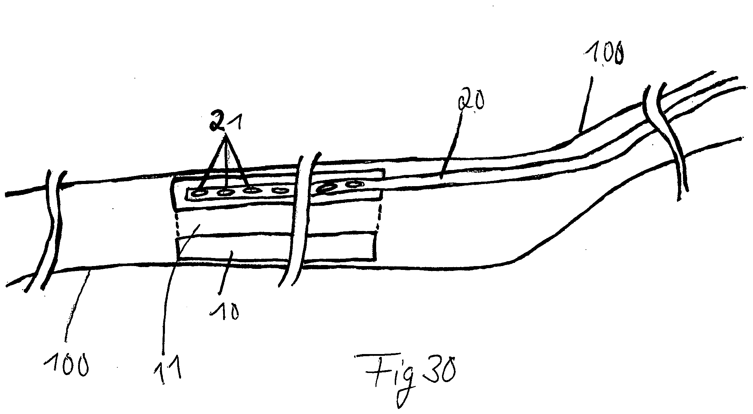

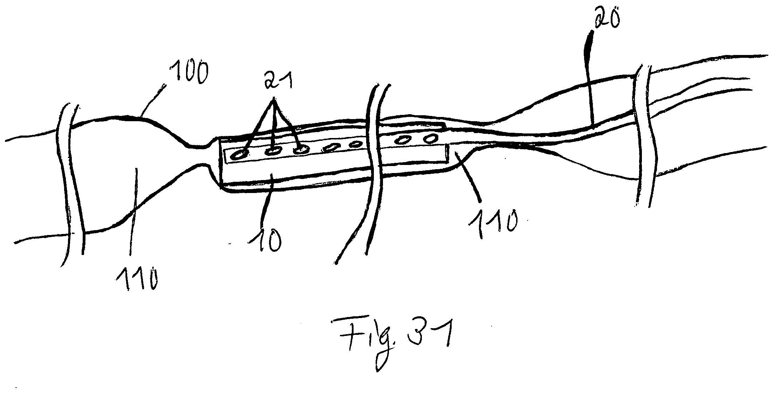

Fig. 30 zeigt eine Querschnittdarstellung eines in einem Darm 100 gelegten Schwamms 10 mit Drainageschlauch 20. Am distalen Ende des Drainageschlauches 20, im Schwamm 10 liegend, befinden sich seitliche Perforationsöffnungen 21. Fig. 31 zeigt einen in einem Darm 100 gelegten Schwamm 10, der unter einem Vakuum kollabiert ist. Hierdurch kollabiert auch das Darmlumen 110 am Schwamm 10 und führt zu einem künstlichen Verschluss des Darmlumen 110. Am Distalende der Drainage 20 befinden sich Perforationsöffnungen 21.

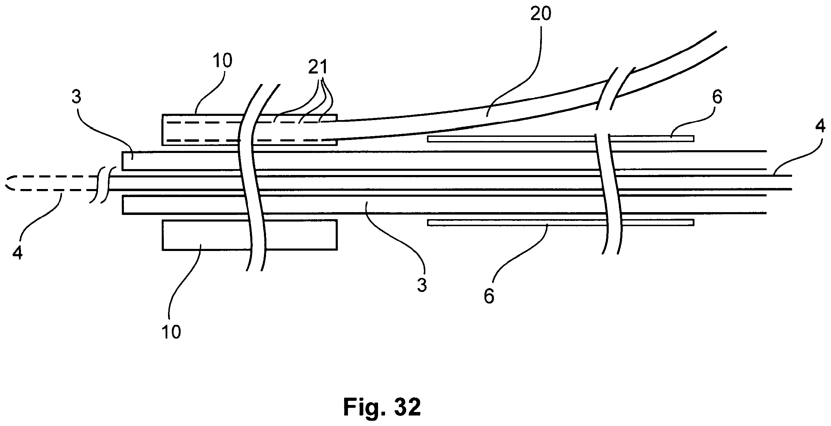

Fig. 32 ist eine Querschnittdarstellung des kompletten Drainagesystems (röhrenförmiger Schwamm 10, Drainageschlauch 20 mit Perforationsöffnungen 21 am Distalende) auf dem distalen Ende eines Endoskops 3 montiert. Das Endoskop 3 liegt in einem Overtube 6. Im Arbeitskanal des Endoskops 3 liegt eine Ernährungssonde 4.

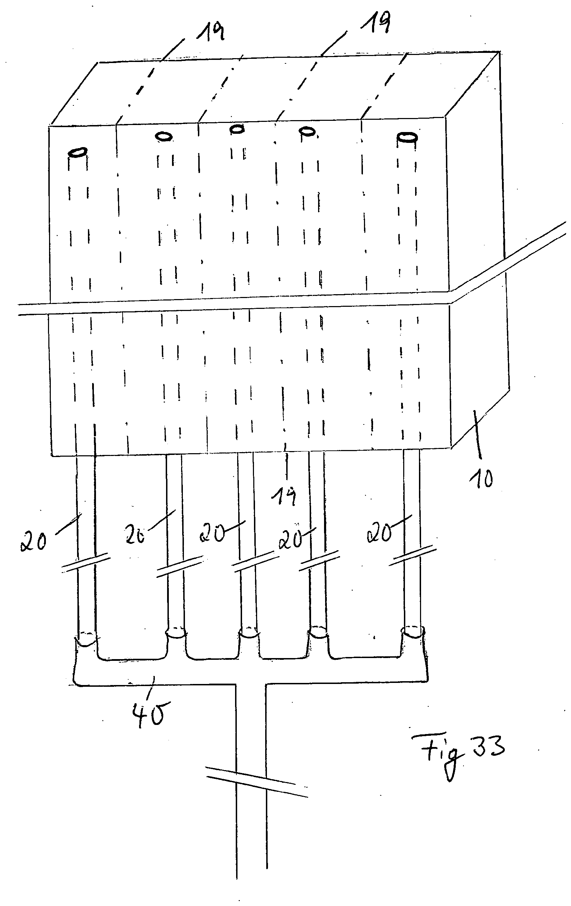

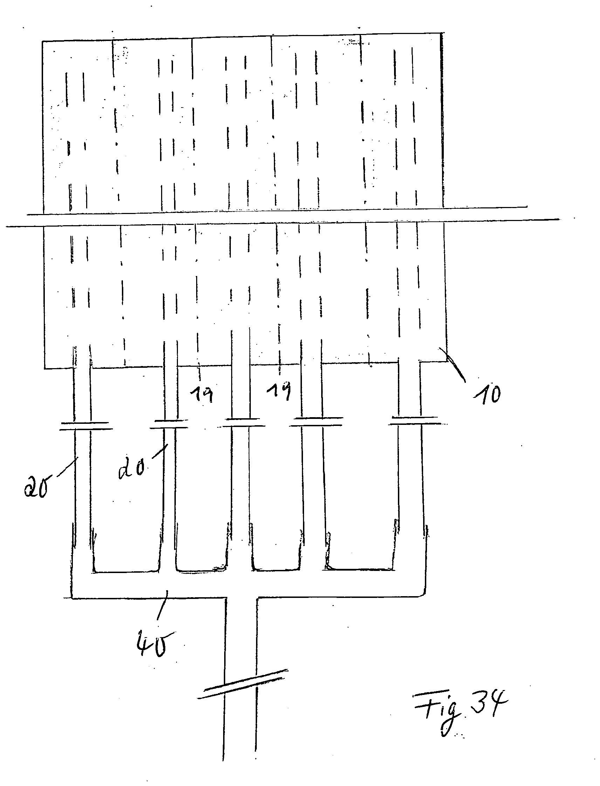

Eine weitere Ausführungsform gemäß der Erfindung ist in Figuren 33 bis 37 gezeigt. Figur 33 ist die Darstellung eines Schwammes 10, in den mehrere am distalen Ende perforierte Drainageschläuche 20 eingebracht wurden. Die Drainageschläuche 20 sind über ein Verbindungselement 40 miteinander verbunden. Der Schwamm ist zwischen den Drainageschläuchen perforiert (Perforationslinie 19). Fig. 34 ist eine Querschnittdarstellung von Figur 33.

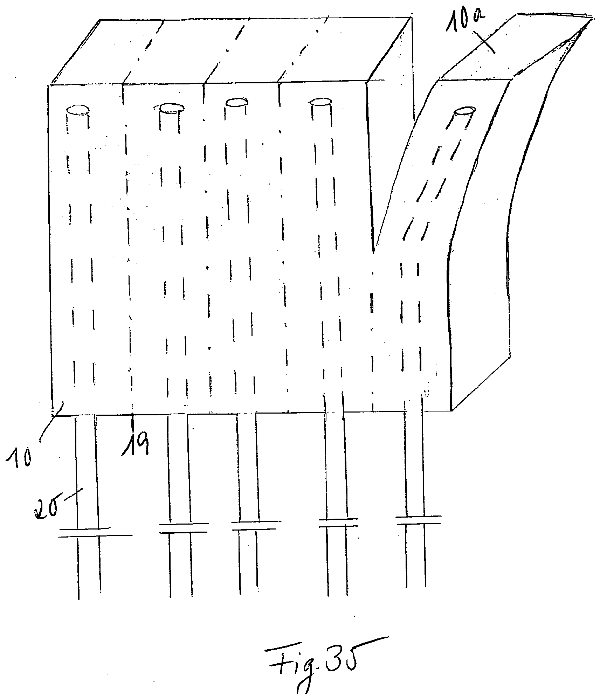

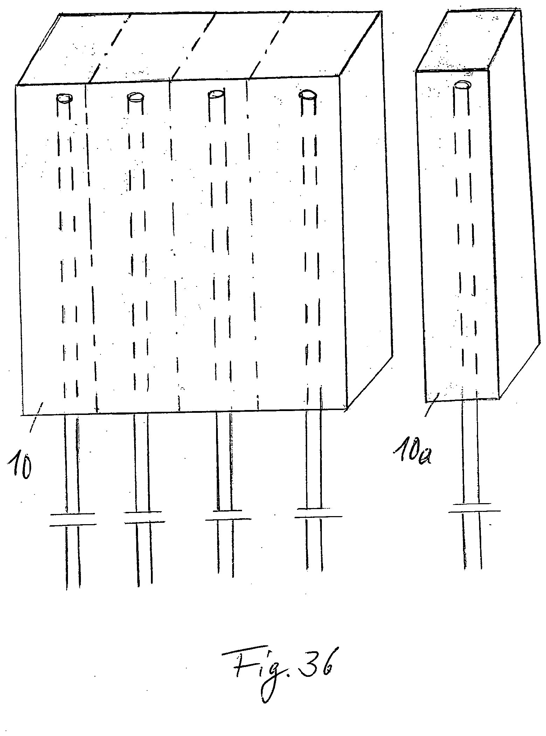

Fig. 35 zeigt die Abtrennung einer Untereinheit 10a vom Schwamm 10. Fig. 36 zeigt die vollständige Trennung einer Untereinheit 10a vom Schwamm 10.

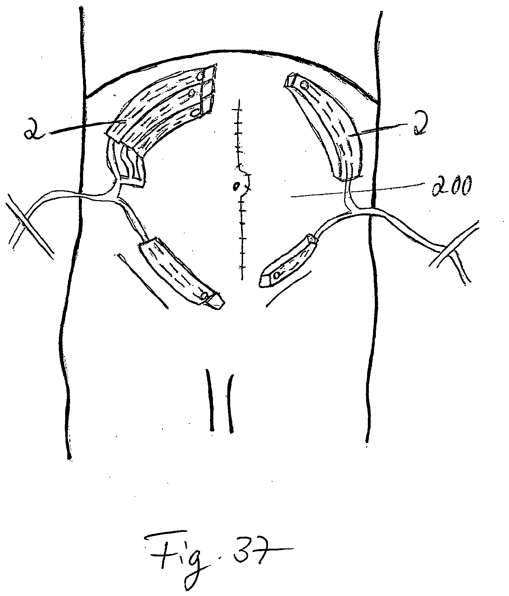

Figur 37 ist eine schematische Darstellung von der Anordnung von Vakuumschwammdrainagen 2 in der Bauchhöhle 200 mit unterschiedlichen Einheitsgrößen.

In den Figuren ist der erfindungsgemäße Kanal in dem Fluidsammelelement mit 11 bezeichnet.

Die unter Bezugnahme auf die Figuren 8 bis 15 erläuterten Merkmale gelten in analoger Weise für das geschlitzte Fluidsammelelement.

Figuren 16 bis 21 illustrieren zwar nur eine Auslassung 17, aber ein erfindungsgemäßer Kanal 11 kann in der erläuterten vorteilhaften Weise vorgesehen sein. Vorteilhaft ist das Fluidsammelelement kappenartig ausgebildet und das Fluidkommunikationselement erstreckt sich nicht entlang der gesamten Länge des Fluidsammelelementes.

Figuren 33 bis 37 illustrieren eine Vielzahl von Fluidkommunikationselementen. Ein erfindungsgemäßer Kanal 11 kann zusätzlich vorgesehen sein. Die Untereinheiten 10a sind bevorzugt jeweils so ausgestaltet wie es unter Bezugnahme auf Figuren 16 bis 21 erläutert ist. In einer weiteren Ausführungsform sind die Untereinheiten 10a eine erfindungsgemäße Vakuumschwammeinheit gemäß der Erfindung.

## Patentansprüche

1. Vakuumschwammsystem (1) mit einer Vakuumschwammeinheit (2) zur Verwendung im menschlichen oder tierischen Körper mit einem Fluidsammelelement (10, 10a, 30) in Form einer Schwammeinheit und einem Fluidkommunikationselement (20) in Form eines Drainageschlauchs, welches wenigstens teilweise in dem Fluidsammelelement (10) angeordnet und fluidleitend mit dem Fluidsammelelement (10, 10a, 30) verbunden ist, wobei das Fluidsammelelement (10, 10a, 30) einen Kanal (11) zum Führen eines Führungselements (3) durch das Fluidsammelelement (10) aufweist, um das Fluidsammelelement entlang des Führungselements zu führen und zu positionieren, und einem Führungselement (3), insbesondere in Form eines Führungsdrahts, und/oder mit einem Versorgungselement (4), insbesondere in Form einer Sondeneinheit oder eines Endoskops, **dadurch gekennzeichnet, daß** das Fluidkommunikationselement (20) in dem Fluidsammelelement (10, 10a, 30) beabstandet von dem Kanal zum Führen des Führungselements (3) und/oder des Versorgungselements (4) angeordnet ist, und eine Vakuumpumpe vorgesehen ist, die mit dem Fluidkommunikationselement (20) fluidleitend verbunden ist und ausgestaltet ist, um einen Unterdruck von bis zu 200 mmHg an das Fluidsammelelement (10) anzulegen.

2. Vakuumschwammsystem (1) nach Anspruch 1, bei der der Kanal (11) eine erste Öffnung (12) als Eingang für das Führungselement (3) und/oder das Versorgungselement (4) und eine zweite Öffnung (13) als Ausgang für das Führungselement (3) und/oder das Versorgungselement (4) aufweist und wobei die erste und die zweite Öffnung (12, 13) auf sich im wesentlichen gegenüberliegenden Oberflächen des Fluidsammelelements (10) angeordnet sind.

3. Vakuumschwammsystem (1) nach wenigstens einem der vorstehenden Ansprüche, bei der das Fluidsammelelement (10) einen Längsschlitz (14) entlang der Längsachse des Fluidsammelelements (10) aufweist, der sich zwischen der Oberfläche des Fluidsammelelements (10) und dem Kanal (11) erstreckt.

4. Vakuumschwammsystem (1) nach wenigstens einem der vorstehenden Ansprüche, bei der das Fluidsammelelement (10) eine Röntgenmarkierung (15) aufweist, die ausgestaltet ist, bei einer Röntgenaufnahme detektiert zu werden.

5. Vakuumschwammsystem (1) nach wenigstens einem der vorstehenden Ansprüche, bei der das Fluidkommunikationselement (20) durch eine Fluidkommunikationselementöffnung (16) in das Fluidsammelelement (10) eingeführt ist und sich entlang der vollständigen Länge des Fluidsammelelements (10) erstreckt.

6. Vakuumschwammsystem (1) nach wenigstens einem der vorstehenden Ansprüche, bei der das Fluidkommunikationselement (20) in demjenigen Bereich, welcher in dem Fluidsammelelement (10) angeordnet ist, eine Vielzahl von Öffnungen (21) aufweist.

7. Vakuumschwammsystem (1) nach wenigstens einem der vorstehenden Ansprüche, bei der ein zusätzliches Fluidsammelelement (30) an dem Fluidkommunikationselement (20) angeordnet ist.

8. Vakuumschwammsystem (1) nach Anspruch 1, bei der das Fluidsammelelement (10) aufgrund mindestens einer Perforation (19) in Fluidsammelelement-Untereinheiten (10a) aufteilbar ist, und bei der in diese Fluidsammelelement-Untereinheiten (10) des Fluidsammelelements eingebrachte Fluidkommunikationselemente (20) über ein Verbindungselement (40) miteinander verbunden sind.

9. Vakuumschwammsystem (1) nach Anspruch 1, das ferner einen Overtube (6) zum Umhüllen der Vakuumschwammeinheit (2), des Führungselements (3) und/oder des Versorgungselements (4) aufweist.

10. Vakuumschwammsystem (1) nach Anspruch 9, bei dem der Overtube (6) entlang seiner Längsachse vollständig geschlitzt ist.

11. Vakuumschwammsystem (1) nach Anspruch 9 oder 10, bei dem der Overtube (6) ein erstes Ende (6a) aufweist, welches konisch ausgestaltet ist, und/oder ein zweites Ende (6b) aufweist, welches trichterförmig ausgestaltet ist.

12. Vakuumschwammsystem (1) nach wenigstens einem der vorhergehenden Ansprüche, welches ferner eine Positionierungshülse (7) und eine Führungshülse (8) aufweist, die gegenüber der Positionierungshülse (7) verschiebbar ausgestaltet ist.

13. Vakuumschwammsystem (1) nach wenigstens einem der Ansprüche 9 bis 12, bei dem das Versorgungselement (4) das Führungselement (3) aufweist.

## Claims

1. A negative-pressure sponge system (1) comprising
- a negative-pressure sponge unit (2) for use in a human or animal body, comprising a fluid collecting element (10, 10a, 30) in the form of a sponge unit and a fluid communication element (20) in the form of a drainage tube, which is at least partially arranged in the fluid collecting element (10) and connected to the fluid collecting element (10, 10a, 30) such that fluid is conducted, wherein the fluid collecting element (10, 10a, 30) comprises a channel (11) for guiding a guide element (3) through the fluid collecting element (10), in order to guide the fluid collecting element along the guide element and into position, and
- a guide element (3), in particular a guide wire, and/or a treatment element (4), in particular a probe unit and/or an endoscope,
**characterised in that** the fluid communication element (20) is placed in the fluid collecting element (10, 10a, 30) at a distance from the channel for guiding the guide element (3) and/or the treatment element (4), and **in that** a vacuum pump is provided which is connected to the fluid communication element (20) in a fluid-conducting manner, and which is designed to apply a negative pressure of up to 200 mmHg to the fluid collecting element (10).

2. The negative-pressure sponge system (1) according to claim 1, wherein the channel (11) has a first opening (12) as an inlet for the guide element (3) and/or the treatment element (4) and a second opening (13) as an outlet for the guide element (3) and/or the treatment element (4), and wherein the first and second openings (12, 13) are located on substantially opposite surfaces of the fluid collecting element (10).

3. The negative-pressure sponge system (1) according to at least one of the preceding claims, wherein the fluid collecting element (10) has a longitudinal slot (14) along the longitudinal axis of the fluid collecting element (10), extending between the surface of the fluid collecting element (10) and the channel (11).

4. The negative-pressure sponge system (1) according to at least one of the preceding claims, wherein the fluid collecting element (10) has an X-ray marking (15) that is designed to be detected during X-ray imaging.

5. The negative-pressure sponge system (1) according to at least one of the preceding claims, wherein the fluid communication element (20) is inserted into the fluid collecting element (10) through a fluid communication element opening (16) and extends along the full length of the fluid collecting element (10).

6. The negative-pressure sponge system (1) according to at least one of the preceding claims, wherein the fluid communication element (20) has a plurality of openings (21) in the area that is located inside the fluid collecting element (10).

7. The negative-pressure sponge system (1) according to at least one of the preceding claims, wherein an additional fluid collecting element (30) is located on the fluid communication element (20).

8. The negative-pressure sponge system (1) according to claim 1, wherein the fluid collecting element (10) can be divided into fluid collecting element sub-units (10a) based on at least one perforation (19), and wherein fluid communication elements (20) inserted into these fluid collecting element sub-units (10a) of the fluid collecting element are connected to each other via a connecting element (40).

9. The negative-pressure sponge system (1) according to claim 1, further comprising an overtube (6) for sheathing the negative-pressure sponge unit (2), the guide element (3) and/or the treatment element (4).

10. The negative-pressure sponge system (1) according to claim 9, wherein the overtube (6) has a slot along the full length of its longitudinal axis.

11. The negative-pressure sponge system (1) according to claim 9 or 10, wherein the overtube (6) has a first end (6a) which has a conical design, and/or has a second end (6b) which is funnel-shaped.

12. The negative-pressure sponge system (1) according to at least one of the preceding claims, which further has a positioning sleeve (7) and a guide sleeve (8) which is designed such that it can be moved towards the positioning sleeve (7).

13. The negative-pressure sponge system (1) according to at least one of claims 9 to 12, wherein the treatment element (4) comprises the guide element (3).

## Revendications

1. Système à éponge pour thérapie par pression négative (1) comportant :
- une unité formant éponge pour thérapie par pression négative (2), conçue pour être utilisée dans un corps humain ou animal, et présentant un élément de recueil de fluide (10, 10a, 30) sous forme d'une unité formant éponge ainsi qu'un élément de communication fluidique (20) sous forme d'un tube de drainage disposé au moins partiellement dans l'élément de recueil de fluide (10) et relié à l'élément de recueil de fluide (10, 10a, 30) de manière à conduire le fluide, ledit élément de recueil de fluide (10, 10a, 30) présentant un canal (11) destiné à guider un élément de guidage (3) à travers l'élément de recueil de fluide (10), pour guider et positionner l'élément de recueil de fluide le long de l'élément de guidage, et
- un élément de guidage (3), notamment sous forme d'un fil de guidage, et/ou un élément d'assistance (4), notamment sous forme d'une unité formant sonde ou d'un endoscope ;
**caractérisé en ce que** l'élément de communication fluidique (20) est disposé dans l'élément de recueil de fluide (10, 10a, 30) en se trouvant à l'écart du canal permettant de guider l'élément de guidage (3) et/ou l'élément d'assistance (4), et **en ce qu'**il est prévu une pompe à vide qui est reliée avec l'élément de communication fluidique (20) de manière à conduire le fluide et est conçue pour appliquer une pression négative allant jusqu'à 200 mmHg sur l'élément de recueil de fluide (10).

2. Système à éponge pour thérapie par pression négative (1) selon la revendication 1, dans lequel le canal (11) présente une première ouverture (12) servant d'entrée pour l'élément de guidage (3) et/ou l'élément d'assistance (4) et une deuxième ouverture (13) servant de sortie pour l'élément de guidage (3) et/ou l'élément d'assistance (4), et dans lequel la première et la deuxième ouverture (12, 13) sont situées sur des surfaces sensiblement opposées de l'élément de recueil de fluide (10).

3. Système à éponge pour thérapie par pression négative (1) selon une au moins des revendications précédentes, dans lequel l'élément de recueil de fluide (10) présente une fente longitudinale (14) sur l'axe longitudinal de l'élément de recueil de fluide (10), laquelle s'étend entre la surface de l'élément de recueil de fluide (10) et le canal (11).

4. Système à éponge pour thérapie par pression négative (1) selon une au moins des revendications précédentes, dans lequel l'élément de recueil de fluide (10) présente un marqueur radiographique (15) conçu pour être détecté dans le cadre d'une radiographie.

5. Système à éponge pour thérapie par pression négative (1) selon une au moins des revendications précédentes, dans lequel l'élément de communication fluidique (20) est introduit dans l'élément de recueil de fluide (10) par une ouverture (16) pour élément de communication fluidique et s'étend sur toute la longueur de l'élément de recueil de fluide (10).

6. Système à éponge pour thérapie par pression négative (1) selon une au moins des revendications précédentes, dans lequel l'élément de communication fluidique (20) présente une pluralité d'ouvertures (21) dans la zone située dans l'élément de recueil de fluide (10).

7. Système à éponge pour thérapie par pression négative (1) selon une au moins des revendications précédentes, dans lequel un élément de recueil de fluide (30) supplémentaire est disposé sur l'élément de communication fluidique (20).

8. Système à éponge pour thérapie par pression négative (1) selon la revendication 1, dans lequel l'élément de recueil de fluide (10) peut être subdivisé en des sous-unités (10a) d'élément de recueil de fluide en raison d'au moins une perforation (19), et dans lequel des éléments de communication fluidique (20) introduits dans lesdites sous-unités (10a) de l'élément de recueil de fluide sont reliés entres eux par un élément de liaison (40).

9. Système à éponge pour thérapie par pression négative (1) selon la revendication 1, présentant en outre un sur-tube (6) destiné à envelopper l'unité formant éponge pour thérapie par pression négative (2), l'élément de guidage (3) et/ou l'élément d'assistance (4).

10. Système à éponge pour thérapie par pression négative (1) selon la revendication 9, dans lequel le sur-tube (6) est fendu sur tout son axe longitudinal.

11. Système à éponge pour thérapie par pression négative (1) selon la revendication 9 ou 10, dans lequel le sur-tube (6) présente une première extrémité (6a) conique et/ou une deuxième extrémité (6b) en forme d'entonnoir.

12. Système à éponge pour thérapie par pression négative (1) selon une au moins des revendications précédentes, présentant en outre une gaine de positionnement (7) et une gaine de guidage (8) conçue coulissante par rapport à la gaine de positionnement (7).

13. Système à éponge pour thérapie par pression négative (1) selon une au moins des revendications 9 à 12, dans lequel l'élément d'assistance (4) présente l'élément de guidage (3).
